(19) Europäisches Patentamt
European Patent Office
Office européen des brevets

(11) **EP 3 532 101 B1**

(12) **EUROPEAN PATENT SPECIFICATION**

(45) Date of publication and mention of the grant of the patent:
**08.12.2021 Bulletin 2021/49**

(51) Int Cl.:
*A61K 39/395* (2006.01)  *A61K 39/00* (2006.01)
*A61K 38/00* (2006.01)  *G01N 33/00* (2006.01)
*C12M 3/00* (2006.01)  *C07K 16/38* (2006.01)

(21) Application number: **17865610.4**

(22) Date of filing: **27.10.2017**

(86) International application number:
**PCT/US2017/058787**

(87) International publication number:
**WO 2018/081578 (03.05.2018 Gazette 2018/18)**

(54) **USE OF ANTIBODIES TO TIMP-2 FOR THE IMPROVEMENT OF RENAL FUNCTION**

VERWENDUNG VON ANTIKÖRPERN GEGEN TIMP-2 ZUR VERBESSERUNG DER NIERENFUNKTION

UTILISATION D'ANTICORPS DIRIGÉS CONTRE TIMP-2 POUR AMÉLIORER LA FONCTION RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2016 US 201662414479 P**

(43) Date of publication of application:
**04.09.2019 Bulletin 2019/36**

(73) Proprietors:
• **Astute Medical, Inc.**
**San Diego, CA 92121 (US)**
• **University of Pittsburgh- Of the Commonwealth System of Higher Education**
**Pittsburgh, PA 15260 (US)**

(72) Inventors:
• **McPHERSON, Paul**
**Encinitas, CA 92024 (US)**
• **KELLUM, John, A.**
**Pittsburgh, PA 15206 (US)**

(74) Representative: **Wilding, James Roger et al**
**Mathys & Squire**
**The Shard**
**32 London Bridge Street**
**London SE1 9SG (GB)**

(56) References cited:
**US-A1- 2015 355 196    US-A1- 2016 281 083**
**US-A1- 2016 281 083    US-A1- 2016 297 893**

**US-A1- 2016 297 893**

• J. A. LOPES ET AL: "The RIFLE and AKIN classifications for acute kidney injury: a critical and comprehensive review", CLINICAL KIDNEY JOURNAL, vol. 6, no. 1, 30 January 2013 (2013-01-30), pages 8-14, XP055165125, ISSN: 2048-8505, DOI: 10.1093/ckj/sfs160
• LOPES ET AL.: 'The RIFLE and AKIN classifications for acute Kidney injury a critical ana comprehensive review' CLIN KIDNEY J. vol. 6, no. 1, 2013, pages 8 - 14, XP055165125
• AMAR ET AL.: 'Potential clinical implications of recent matrix metalloproteinase inhibitor design strategies' EXPERT REV PROTEOMICS vol. 12, no. 5, 2015, pages 445 - 7, XP055484646
• SHIBA ET AL.: 'Chronic kidney disease and heart failure - Bidirectional close link and common therapeutic goal' J CARDIOL. vol. 57, no. 1, 2011, pages 8 - 17, XP027576265
• YAN ET AL.: 'Expression of MMP-2 and TIMP-1 in Renal Tissue of Patients with Chronic Active Antibody-mediated Renal Graft Rejection' DIAGN PATHOL. vol. 7, no. 141, 2012, pages 1 - 6, XP021137264
• OSTERMANN ET AL.: 'Acute kidney injury 2016: diagnosis and diagnostic workup' CRIT CARE. vol. 20, no. 1, September 2016, page 299, XP055484647

**Description**

BACKGROUND

Metalloproteinase inhibitor 2 (human precursor Swiss-Prot P16035, also known as "Tissue inhibitor of metalloproteinases 2" and "TIMP2") is a secreted protein which complexes with metalloproteinases and irreversibly inactivates them by binding to their catalytic zinc cofactor. TIMP2 is known to act on MMP-1, MMP-2, MMP-3, MMP-7, MMP-8, MMP-9, MMP-10, MMP-13, MMP-14, MMP-15, MMP-16 and MMP-19. TIMP2 reportedly suppresses the proliferation of endothelial cells. As a result, the encoded protein has been suggested to have a role in the maintenance of tissue homeostasis by suppressing the proliferation of quiescent tissues in response to angiogenic factors, and by inhibiting protease activity in tissues undergoing remodeling.

In addition, WO2010/048346 and WO2011/075744 describe the use of TIMP2 for evaluating the renal status of a subject both individually and in multimarker panels. In particular, TIMP2 levels measured by immunoassay are shown to correlate to risk stratification, diagnosis, staging, prognosis, classifying and monitoring of renal status.

Acute kidney injury (AKI) is due to a variety of conditions and has serious consequences. AKI is defined as any of the following:

Increase in SCr by $\geq$0.3 mg/dl ($\geq$26.5 $\mu$mol/l) within 48 hours; or
Increase in SCr to $\geq$1.5 times baseline, which is known or presumed to have occurred within the prior 7 days; or
Urine volume <0.5 ml/kg/h for 6 hours.

AKI is staged for severity according to the following criteria:

| Stage | Serum creatinine | Urine output |
|---|---|---|
| 1 | 1.5-1.9 times baseline; or $\geq$0.3 mg/dl ($\geq$26.5 mmol/l) increase | <0.5 ml/kg/h for 6-12 hours |
| 2 | 2.0-2.9 times baseline | <0.5 ml/kg/h for >12 hours |
| 3 | 3.0 times baseline; or initiation of renal replacement therapy; or Increase in serum creatinine to $\geq$4.0 mg/dl ($\geq$353.6 mmol/l); or in patients <18 years, decrease in eGFR to <35 ml/min per 1.73 m$^2$ | <0.5 ml/kg/h for >12 hours; or anuria for $\geq$12 hours |

Importantly, by defining the syndrome of acute changes in renal function more broadly, RIFLE criteria move beyond ARF. The concept of AKI, as defined by RIFLE criteria, includes other, less severe conditions. Bellomo et al., Crit Care. 8(4):R204-12, 2004, describes the RIFLE criteria:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

"Loss": persistent need for renal replacement therapy for more than four weeks.

"ESRD": end stage renal disease-the need for dialysis for more than 3 months.

As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.

It is widely recognized that AKI leads to high morbidity and mortality in hospitalized patients, and there is an urgent need for effective therapy. Except for a few isolated studies, the vast majority of animal and clinical studies have yet to demonstrate conclusively the benefit of a pharmacologic treatment of AKI. Jo et al., Clin J Am Soc Nephrol 2: 356-365, 2007.

**SUMMARY**

It is an object of the disclosure to methods for the treatment of subjects having, or at risk of, renal injuries. Specifically, the administration of antibodies directed to Metalloproteinase inhibitor 2 (TIMP-2) are shown herein to improve kidney histology scores in a model of sepsis-induced AKI.

The present invention is defined in the appended claims. Thus, in a first aspect, the prevent invention provides an antibody that specifically binds Metalloproteinase inhibitor 2 (TIMP-2) for use in treating a subject having acute kidney injury or identified as being at increased risk of acute kidney injury, the use comprising administering an effective amount of the antibody to the subject.

In certain embodiments, the administration of the anti-TIMP-2 antibody is performed optionally in association with one or more further therapeutic agents or therapeutic procedures indicated for the improvement of kidney function, in an amount sufficient to improve kidney function. Suitable additional treatment modalities are described in detail hereinafter.

In certain embodiments a subject in need thereof is a subject having chronic kidney disease (CKD) or that exhibits one or more symptoms of CKD. In other embodiments, a subject in need thereof is a subject having acute kidney injury (AKI) or that exhibits one or more symptoms of AKI.

In still other embodiments, a subject in need thereof is a subject identified as being at increased risk of AKI. By way of example, such a subject may be identified as being at increased risk based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, or reduced renal function. In certain embodiments, the subject is characterized as having diabetic nephropathy (DN) or exhibiting one or more symptoms of DN.

Alternatively, or in addition, such a subject may be identified as being at increased risk based on the subject undergoing or having undergone major vascular surgery, coronary artery bypass, or other cardiac surgery, and/or has received one or more of NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

In certain embodiments such a subject may be identified as being at increased risk based on a biomarker result. Preferably, the biomarker result comprises one or more of a measured urinary TIMP-2 concentration and a measured urinary Insulin-like growth factor-binding protein 7 (IGFBP7) concentration, and most preferably a combined result calculated from a measured urinary TIMP-2 concentration and a measured urinary IGFBP7 concentration, such as a [TIMP-2]x[IGFBP7] result.

In certain embodiments, the subject is characterized as at or below AKIN stage 1; the subject is characterized as at or below AKIN stage 2, or the subject is characterized as at or below AKIN stage 3.

In various embodiments, administration of the anti-TIMP-2 antibody, alone or with the optional treatment modalities, results in an improvement in estimated glomerular filtration rate (eGFR) of the subject.

In various embodiments, administration of the anti-TIMP-2 antibody, alone or with the optional treatment modalities, reduces the level of serum creatinine in the subject.

In various embodiments, administration of the anti-TIMP-2 antibody is parenteral, such as intravenous, intraarterial, or subcutaneous.

In various embodiments, administration of the anti-TIMP-2 antibody is an IgG, an Fab fragment, an F(ab')2, or an scFv. This list is not meant to be limiting. In certain embodiments, the anti-TIMP-2 antibody is humanized or fully human.

In certain embodiments, the one or more further therapeutic agents or therapeutic procedures indicated for the improvement of kidney function comprise one or more treatments selected from the group consisting of renal replacement therapy, management of fluid overload, administration of a caspase inhibitor, administration of minocycline, administration of a Poly ADP-ribose polymerase inhibitor, administration of an iron chelator, administration of a treatment for sepsis in a subject in need thereof, administration of insulin, administration of erythropoietin, and administration of a vasodilator.

The details of one or more embodiments of the disclosure are set forth in the accompanying drawings and the description below. Other features, objects, and advantages of the disclosure will be apparent from the description and drawings, and from the claims.

## BRIEF DESCRIPTION OF THE DRAWINGS

The accompanying drawings, which are incorporated in and constitute a part of this specification, illustrate several embodiments of the invention and together with the description serve to explain the principles of the invention.

FIG. 1 shows kidney tissue injury as measured by histology in a mouse cecal ligation and puncture sepsis model following treatment with anti-TIMP2 as compared to untreated sepsis animals.

FIG. 2 shows kidney function as measured by serum creatinine in a mouse cecal ligation and puncture sepsis model following treatment with anti-TIMP2 as compared to untreated sepsis animals.

## DETAILED DESCRIPTION

Definitions

As used herein, the terms "Metalloproteinase inhibitor 2" and "TIMP-2" refer to one or more polypeptides present in a biological sample that are derived from the Metalloproteinase inhibitor 2 precursor (human precursor: Swiss-Prot P16035 (SEQ ID NO: 10)).

```
          10         20         30         40         50         60
   MGAAARTLRL ALGLLLLATL LRPADACSCS PVHPQQAFCN ADVVIRAKAV SEKEVDSGND

          70         80         90        100        110        120
   IYGNPIKRIQ YEIKQIKMFK GPEKDIEFIY TAPSSAVCGV SLDVGGKKEY LIAGKAEGDG

         130        140        150        160        170        180
   KMHITLCDFI VPWDTLSTTQ KKSLNHRYQM GCECKITRCP MIPCYISSPD ECLWMDWVTE

         190        200        210        220
   KNINGHQAKF FACIKRSDGS CAWYRGAAPP KQEFLDIEDP
```

The following domains have been identified in Metalloproteinase inhibitor 2:

| Residues | Length | Domain ID |
| --- | --- | --- |
| 1-26 | 26 | Signal peptide |
| 27-220 | 194 | Metalloproteinase inhibitor 2 |

Unless specifically noted otherwise herein, the definitions of the terms used are standard definitions used in the art of pharmaceutical sciences. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

The term "subject" as used herein refers to a human or non-human organism. Thus, the methods and compositions described herein are applicable to both human and veterinary disease. Further, while a subject is preferably a living organism, the disclosure described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present disclosure , optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Certain therapeutic antibodies are IgG antibodies. The term "IgG" as used herein is meant a polypeptide belonging to the class of antibodies that are substantially encoded by a recognized immunoglobulin gamma gene. In humans this class comprises IgG1, IgG2, IgG3, and IgG4. In mice this class comprises IgG1, IgG2a, IgG2b, IgG3. The known Ig domains in the IgG class of antibodies are VH, $C\gamma1$, $C\gamma2$, $C\gamma3$, VL, and CL. IgG is the preferred class for therapeutic antibodies for several practical reasons. IgG antibodies are stable, easily purified, and able to be stored under conditions that are practical for pharmaceutical supply chains. *In vivo* they have a long biological half-life that is not just a function of their size but is also a result of their interaction with the so-called Fc receptor (or FcRn). This receptor seems to protect IgG from catabolism within cells and recycles it back to the plasma.

Antibodies are immunological proteins that bind a specific antigen. In most mammals, including humans and mice, antibodies are constructed from paired heavy and light polypeptide chains. The light and heavy chain variable regions show significant sequence diversity between antibodies, and are responsible for binding the target antigen. Each chain is made up of individual immunoglobulin (Ig) domains, and thus the generic term immunoglobulin is used for such proteins.

The present disclosure includes anti-TIMP-2 antigen-binding fragments and methods of use thereof. As used herein, unless otherwise indicated, "antibody fragment" or "antigen-binding fragment" refers to antigen-binding fragments of antibodies, *i.e.* antibody fragments that retain the ability to bind specifically to the antigen bound by the full-length antibody, *e.g.* fragments that retain one or more CDR regions. Examples of antigen-binding fragments include, but are not limited to, Fab, Fab', F(ab')$_2$, and Fv fragments; diabodies; linear antibodies; single-chain antibody molecules, *e.g.,* sc-Fv; nanobodies; and multispecific antibodies formed from antibody fragments (e.g., bispecific antibodies, etc.).

The present disclosure includes anti-TIMP-2 Fab fragments and methods of use thereof. A "Fab fragment" is comprised of one light chain and the $C_H1$ and variable regions of one heavy chain. The heavy chain of a Fab molecule cannot form a disulfide bond with another heavy chain molecule. An "Fab fragment" can be the product of papain cleavage of an antibody.

The present disclosure includes anti-TIMP-2 antibodies and antigen-binding fragments thereof which comprise an Fc region and methods of use thereof. An "Fc" region contains two heavy chain fragments comprising the CH1 and CH2 domains of an antibody. The two heavy chain fragments are held together by two or more disulfide bonds and by hydrophobic interactions of the CH3 domains.

The present disclosure includes anti-TIMP-2 Fab' fragments and methods of use thereof. A "Fab' fragment" contains one light chain and a portion or fragment of one heavy chain that contains the $V_H$ domain and the $C_H1$ domain and also the region between the $C_H1$ and $C_H2$ domains, such that an interchain disulfide bond can be formed between the two heavy chains of two Fab' fragments to form a F(ab')$_2$ molecule.

The present disclosure includes anti-TIMP-2 F(ab')2 fragments and methods of use thereof. A "F(ab')$_2$ fragment" contains two light chains and two heavy chains containing a portion of the constant region between the $C_{H1}$ and $C_{H2}$ domains, such that an interchain disulfide bond is formed between the two heavy chains. A F(ab')$_2$ fragment thus is composed of two Fab' fragments that are held together by a disulfide bond between the two heavy chains. A "F(ab')$_2$ fragment" can be the product of pepsin cleavage of an antibody.

The present disclosure includes anti-TIMP-2 Fv fragments and methods of use thereof. The "Fv region" comprises the variable regions from both the heavy and light chains, but lacks the constant regions.

The present disclosure includes anti-TIMP-2 scFv fragments and methods of use thereof. The term "single-chain Fv" or "scFv" antibody refers to antibody fragments comprising the $V_H$ and $V_L$ domains of an antibody, wherein these domains are present in a single polypeptide chain. Generally, the Fv polypeptide further comprises a polypeptide linker between the $V_H$ and $V_L$ domains which enables the scFv to form the desired structure for antigen-binding. For a review of scFv, see Pluckthun (1994) THE PHARMACOLOGY OF MONOCLONAL ANTIBODIES, vol. 113, Rosenburg and Moore eds. Springer-Verlag, New York, pp. 269-315. See also, International Patent Application Publication No. WO 88/01649 and U.S. Pat. Nos. 4,946, 778 and 5,260,203.

The present disclosure includes anti-TIMP-2 domain antibodies and methods of use thereof. A "domain antibody" is an immunologically functional immunoglobulin fragment containing only the variable region of a heavy chain or the variable region of a light chain. In some instances, two or more $V_H$ regions are covalently joined with a peptide linker to create a bivalent domain antibody. The two $V_H$ regions of a bivalent domain antibody may target the same or different antigens.

The present disclosure includes anti-TIMP-2 bivalent antibodies and methods of use thereof. A "bivalent antibody" comprises two antigen-binding sites. In some instances, the two binding sites have the same antigen specificities. However, bivalent antibodies may be bispecific (see below).

The present disclosure includes anti-TIMP-2 camelized single domain antibodies and methods of use thereof. In certain embodiments, antibodies herein also include camelized single domain antibodies. *See, e.g.,* Muyldermans et al. (2001) Trends Biochem. Sci. 26:230; Reichmann et al. (1999) J. Immunol. Methods 231:25; WO 94/04678; WO 94/25591; U.S. Pat. No. 6,005,079). In one instance, the present disclosure provides single domain antibodies comprising two $V_H$ domains with modifications such that single domain antibodies are formed.

The present disclosure includes anti-TIMP-2 diabodies and methods of use thereof. As used herein, the term "diabodies" refers to small antibody fragments with two antigen-binding sites, which fragments comprise a heavy chain variable domain ($V_H$) connected to a light chain variable domain ($V_L$) in the same polypeptide chain ($V_H$-$V_L$ or $V_L$-$V_H$). By using a linker that is too short to allow pairing between the two domains on the same chain, the domains are forced to pair with the complementary domains of another chain and create two antigen-binding sites. Diabodies are described more fully in, *e.g.,* EP 404,097; WO 93/11161; and Holliger et al. (1993) Proc. Natl. Acad. Sci. USA 90: 6444-6448. For a review of engineered antibody variants generally see Holliger and Hudson (2005) Nat. Biotechnol. 23:1126-1136.

The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$ .

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

Antibodies of the disclosure may be further characterized by epitope mapping, so that antibodies and epitopes may be selected that have the greatest clinical utility in the immunoassays described herein. The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents. Preferably, an epitope is targeted which is present on the target molecule, but is partially or totally absent on non-target molecules.

In some embodiments, the antibody scaffold can be a mixture of sequences from different species. As such, if the antibody is an antibody, such antibody may be a chimeric antibody and/or a humanized antibody. In general, both "chimeric antibodies" and "humanized antibodies" refer to antibodies that combine regions from more than one species. For example, "chimeric antibodies" traditionally comprise variable region(s) from a mouse (or rat, in some cases) and the constant region(s) from a human. "Humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536. "Backmutation" of selected acceptor framework residues to the corresponding donor residues is often required to regain affinity that is lost in the initial grafted construct (U.S. Pat. No. 5,530,101; U.S. Pat. No. 5,585,089; U.S. Pat. No. 5,693,761; U.S. Pat. No. 5,693,762; U.S. Pat. No. 6,180,370; U.S. Pat. No. 5,859,205; U.S. Pat. No. 5,821,337; U.S. Pat. No. 6,054,297; U.S. Pat. No. 6,407,213). The humanized antibody optimally also will comprise at least a portion of an immunoglobulin constant region, typically that of a human immunoglobulin, and thus will typically comprise a human Fc region. Humanized antibodies can also be generated using mice with a genetically engineered immune system. Roque et al., 2004, Biotechnol. Prog. 20:639-654. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein). Humanization methods include but are not limited to methods described in Jones et al., 1986, Nature 321:522-525; Riechmann et al., 1988; Nature 332:323-329; Verhoeyen et al., 1988, Science, 239:1534-1536; Queen et al., 1989, Proc Natl Acad Sci, USA 86:10029-33; He et al., 1998, J. Immunol. 160: 1029-1035; Carter et al., 1992, Proc Natl Acad Sci USA 89:4285-9, Presta et al., 1997, Cancer Res.57(20):4593-9; Gorman et al., 1991, Proc. Natl. Acad. Sci. USA 88:4181-4185; O'Connor et al., 1998, Protein Eng 11:321-8. Humanization or other methods of reducing the immunogenicity of nonhuman antibody variable regions may include resurfacing methods, as described for example in Roguska et al., 1994, Proc. Natl. Acad. Sci. USA 91:969-973. In one embodiment, the parent antibody has been affinity matured, as is known in the art. Structure-based methods may be employed for humanization and affinity maturation, for example as described in U.S. Ser. No. 11/004,590. Selection based methods may be employed to humanize and/or affinity mature antibody variable regions, including but not limited to methods described in Wu et al., 1999, J. Mol. Biol. 294:151-162; Baca et al., 1997, J. Biol. Chem. 272(16):10678-10684; Rosok et al., 1996, J. Biol. Chem. 271(37): 22611-22618; Rader et al., 1998, Proc. Natl. Acad. Sci. USA 95: 8910-8915; Krauss et al., 2003, Protein Engineering 16(10):753-759. Other humanization methods may involve the grafting of only parts of the CDRs, including but not limited to methods described in U.S. Ser. No. 09/810,502; Tan et al., 2002, J. Immunol. 169:1119-1125; De Pascalis et al., 2002, J. Immunol. 169:3076-3084.

In one embodiment, the antibody is a fully human antibody. "Fully human antibody" or "complete human antibody" refers to a human antibody having the gene sequence of an antibody derived from a human chromosome. Fully human antibodies may be obtained, for example, using transgenic mice (Bruggemann et al., 1997, Curr Opin Biotechnol 8:455-458) or human antibody libraries coupled with selection methods (Griffiths et al., 1998, Curr Opin Biotechnol 9:102-108).

Production of Antibodies

Monoclonal antibody preparations can be produced using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., ANTIBODIES: A LABORATORY MANUAL, (Cold Spring Harbor Laboratory Press, 2nd ed. 1988); Hammerling, et al., in: MONOCLONAL ANTIBODIES AND T-CELL HYBRIDOMAS, pp. 563-681 (Elsevier, N.Y., 1981).

The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Monoclonal antibodies derived from animals other than rats and mice offer unique advantages. Many protein targets relevant to signal transduction and disease are highly conserved between mice, rats and humans, and can therefore be recognized as self-antigens by a mouse or rat host, making them less immunogenic. This problem may be avoided when using rabbit as a host animal. See, e.g., Rossi et al., Am. J. Clin. Pathol., 124, 295-302, 2005.

Methods for producing and screening for specific antibodies using hybridoma technology are routine and well known in the art. In a non-limiting example, mice can be immunized with an antigen of interest or a cell expressing such an antigen. Once an immune response is detected, e.g., antibodies specific for the antigen are detected in the mouse serum, the mouse spleen is harvested and splenocytes isolated. The splenocytes are then fused by well known techniques to any suitable myeloma cells. Hybridomas are selected and cloned by limiting dilution. The hybridoma clones are then assayed by methods known in the art for cells that secrete antibodies capable of binding the antigen. Ascites fluid, which generally contains high levels of antibodies, can be generated by inoculating mice intraperitoneally with positive hybridoma clones.

Adjuvants that can be used in the methods of antibody generation include, but are not limited to, protein adjuvants; bacterial adjuvants, e.g., whole bacteria (BCG, Corynebacterium parvum, Salmonella minnesota) and bacterial components including cell wall skeleton, trehalose dimycolate, monophosphoryl lipid A, methanol extractable residue (MER) of tubercle bacillus, complete or incomplete Freund's adjuvant; viral adjuvants; chemical adjuvants, e.g., aluminum hydroxide, iodoacetate and cholesteryl hemisuccinateor; naked DNA adjuvants. Other adjuvants that can be used in the methods of the disclosure include, Cholera toxin, paropox proteins, MF-59 (Chiron Corporation; See also Bieg et al. (1999) "GAD65 And Insulin B Chain Peptide (9-23) Are Not Primary Autoantigens In The Type 1 Diabetes Syndrome Of The BB Rat," Autoimmunity, 31(1):15-24, MPL® (Corixa Corporation; See also Lodmell et al. (2000) "DNA Vaccination Of Mice Against Rabies Virus: Effects Of The Route Of Vaccination And The Adjuvant Monophosphoryl Lipid A (MPL)," Vaccine, 18: 1059-1066; Johnson et al. (1999) "3-O-Desacyl Monophosphoryl Lipid A Derivatives: Synthesis And Immunostimulant Activities," Journal of Medicinal Chemistry, 42: 4640-4649; Baldridge et al. (1999) "Monophosphoryl Lipid A (MPL) Formulations For The Next Generation Of Vaccines," Methods, 19: 103-107, RC-529 adjuvant (Corixa Corporation; the lead compound from Corixa's aminoalkyl glucosaminide 4-phosphate (AGP) chemical library, see also www.corixa.com), and DETOX™ adjuvant (Corixa Corporation; DETOX™ adjuvant includes MPL® adjuvant (monophosphoryl lipid A) and mycobacterial cell wall skeleton; See also Eton et al. (1998) "Active Immunotherapy With Ultraviolet B-Irradiated Autologous Whole Melanoma Cells Plus DETOX In Patients With Metastatic Melanoma," Clin. Cancer Res. 4(3):619-627; and Gupta et al. (1995) "Adjuvants For Human Vaccines-Current Status, Problems And Future Prospects," Vaccine, 13(14): 1263-1276).

Numerous publications discuss the use of phage display technology to produce and screen libraries of polypeptides for binding to a selected analyte. See, e.g, Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g., U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

Antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express human antibodies. The transgenic mice are immunized using conventional methodologies with a selected antigen, e.g., all or a portion of a polypeptide of the disclosure . Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg et al. (1995) "Human Antibodies From Transgenic Mice," Int. Rev. Immunol. 13:65-93.

For a detailed discussion of this technology for producing human antibodies and human monoclonal antibodies and protocols for producing such antibodies, see, e.g., International Publication Nos. WO 98/24893, WO 96/34096, and WO 96/33735; and U.S. Pat. Nos. 5,413,923, 5,625,126, 5,633,425, 5,569,825, 5,661,016, 5,545,806, 5,814,318, and 5,939,598.

In addition, companies such as Abgenix, Inc. (Freemont, Calif.) and Medarex (Princeton, N.J.) can be engaged to provide human antibodies directed against a selected antigen using technology similar to that described above.

Recombinant Expression of Antibodies

Once a nucleic acid sequence encoding an antibody of the disclosure has been obtained, the vector for the production of the antibody may be produced by recombinant DNA technology using techniques well known in the art. Methods which are well known to those skilled in the art can be used to construct expression vectors containing the antibody coding sequences and appropriate transcriptional and translational control signals. These methods include, for example, in vitro recombinant DNA techniques, synthetic techniques, and in vivo genetic recombination. (See, for example, the techniques described in Sambrook et al, 1990, MOLECULAR CLONING, A LABORATORY MANUAL, 2d Ed., Cold Spring Harbor Laboratory, Cold Spring Harbor, N.Y. and Ausubel et al. eds., 1998, CURRENT PROTOCOLS IN MOLECULAR BIOLOGY, John Wiley & Sons, NY).

An expression vector comprising the nucleotide sequence of an antibody can be transferred to a host cell by conventional techniques (e.g., electroporation, liposomal transfection, and calcium phosphate precipitation) and the transfected cells are then cultured by conventional techniques to produce the antibody of the disclosure. In specific instances, the expression of the antibody is regulated by a constitutive, an inducible or a tissue, specific promoter.

Eukaryotic and prokaryotic host cells, including mammalian cells as hosts for expression of the antibodies or fragments or immunoglobulin chains disclosed herein are well known in the art and include many immortalized cell lines available from the American Type Culture Collection (ATCC). These include, *inter alia,* Chinese hamster ovary (CHO) cells, NS0, SP2 cells, HeLa cells, baby hamster kidney (BHK) cells, monkey kidney cells (COS), human hepatocellular carcinoma cells (*e.g.,* Hep G2), A549 cells, 3T3 cells, HEK-293 cells and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. Fungal cells include yeast and filamentous fungus cells including, for example, *Pichia pastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia* sp., *Saccharomyces cerevisiae, Saccharomyces* sp., *Hansenula polymorpha, Kluyveromyces sp., Kluyveromyces lactis, Candida albicans, Aspergillus nidulans, Aspergillus niger, Aspergillus oryzae, Trichoderma reesei, Chrysosporium lucknowense, Fusarium* sp., *Fusarium gramineum, Fusarium venenatum, Physcomitrella patens* and *Neurospora crassa. Pichia sp.,* any *Saccharomyces sp., Hansenula polymorpha,* any *Kluyveromyces sp., Candida albicans,* any *Aspergillus sp., Trichoderma reesei, Chrysosporium lucknowense,* any *Fusarium sp., Yarrowia lipolytica,* and *Neurospora crassa.* When recombinant expression vectors encoding the heavy chain or antigen-binding portion or fragment thereof, the light chain and/or antigen-binding fragment thereof are introduced into mammalian host cells, the antibodies are produced by culturing the host cells for a period of time sufficient to allow for expression of the antibody or fragment or chain in the host cells or secretion of the into the culture medium in which the host cells are grown.

A variety of host-expression vector systems may be utilized to express the antibodies of the disclosure . Such host-expression systems represent vehicles by which the coding sequences of the antibodies may be produced and subsequently purified, but also represent cells which may, when transformed or transfected with the appropriate nucleotide coding sequences, express the antibodies of the disclosure in situ. These include, but are not limited to, microorganisms such as bacteria (e.g., E. coli and B. subtilis) transformed with recombinant bacteriophage DNA, plasmid DNA or cosmid DNA expression vectors containing immunoglobulin coding sequences; yeast (e.g., Saccharomyces pichia) transformed with recombinant yeast expression vectors containing immunoglobulin coding sequences; insect cell systems infected with recombinant virus expression vectors (e.g., baculovirus) containing the immunoglobulin coding sequences; plant cell systems infected with recombinant virus expression vectors (e.g., cauliflower mosaic virus (CaMV) and tobacco mosaic virus (TMV)) or transformed with recombinant plasmid expression vectors (e.g., Ti plasmid) containing immunoglobulin coding sequences; or mammalian cell systems (e.g., COS, CHO, BHK, 293, 293T, 3T3 cells, lymphotic cells (see U.S. Pat. No. 5,807,715), Per C.6 cells (rat retinal cells developed by Crucell)) harboring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the antibody being expressed. For example, when a large quantity of such a protein is to be produced, for the generation of pharmaceutical compositions of an antibody, vectors which direct the expression of high levels of fusion protein products that are readily purified may be desirable. Such vectors include, but are not limited to, the E. coli expression vector pUR278 (Ruther et al. (1983) "Easy Identification Of cDNA Clones," EMBO J. 2:1791-1794), in which the antibody coding sequence may be ligated individually into the vector in frame with the lac Z coding region so that a fusion protein is produced; pIN vectors (Inouye et al. (1985) "Up-Promoter Mutations In The Lpp Gene Of Escherichia coli," Nucleic Acids Res. 13:3101-3110; Van Heeke et al. (1989) "Expression Of Human Asparagine Synthetase In Escherichia coli," J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free gluta-thione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in Spodoptera frugiperda cells. The antibody coding sequence may be cloned individually into non-essential regions (e.g., the polyhedrin gene) of the virus and placed under control of an AcNPV promoter (e.g., the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing the immunoglobulin molecule in infected hosts. (see e.g., see Logan et al. (1984) "Adenovirus Tripartite Leader Sequence Enhances Translation Of mRNAs Late After Infection," Proc. Natl. Acad. Sci. (U.S.A.) 81:3655-3659). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bitter et al. (1987) "Expression And Secretion Vectors For Yeast," Methods in Enzymol. 153:516-544).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 293T, 3T3, WI38, BT483, Hs578T, HTB2, BT20 and T47D, CRL7030 and Hs578Bst.

For long-term, high-yield production of recombinant proteins, stable expression is preferred. For example, cell lines which stably express an antibody of the disclosure may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibodies of the disclosure. Such engineered cell lines may be particularly useful in screening and evaluation of compounds that interact directly or indirectly with the antibodies of the disclosure .

A number of selection systems may be used, including but not limited to the herpes simplex virus thymidine kinase (Wigler et al. (1977) "Transfer Of Purified Herpes Virus Thymidine Kinase Gene To Cultured Mouse Cells," Cell 11:223-232), hypoxanthine-guanine phosphoribosyltransferase (Szybalska et al. (1962) "Genetics Of Human Cess Line. IV. DNA-Mediated Heritable Transformation Of A Biochemical Trait," Proc. Natl. Acad. Sci. (U.S.A.) 48:2026-2034), and adenine phosphoribosyltransferase (Lowy et al. (1980) "Isolation Of Transforming DNA: Cloning The Hamster Aprt Gene," Cell 22:817-823) genes can be employed in tk-, hgprt- or aprt- cells, respectively. Also, antimetabolite resistance can be used as the basis of selection for the following genes: dhfr, which confers resistance to methotrexate (Wigler et al. (1980) "Transformation Of Mammalian Cells With An Amplfiable Dominant-Acting Gene," Proc. Natl. Acad. Sci. (U.S.A.) 77:3567-3570; O'Hare et al. (1981) "Transformation Of Mouse Fibroblasts To Methotrexate Resistance By A Recombinant Plasmid Expressing A Prokaryotic Dihydrofolate Reductase," Proc. Natl. Acad. Sci. (U.S.A.) 78:1527-1531); gpt, which confers resistance to mycophenolic acid (Mulligan et al. (1981) "Selection For Animal Cells That Express The Escherichia coli Gene Coding For Xanthine-Guanine Phosphoribosyltransferase," Proc. Natl. Acad. Sci. (U.S.A.) 78:2072-2076); neo, which confers resistance to the aminoglycoside G-418 (Tachibana et al. (1991) "Altered Reactivity Of Immunoglobutin Produced By Human-Human Hybridoma Cells Transfected By pSV2-Neo Gene," Cytotechnology 6(3):219-226; Tolstoshev (1993) "Gene Therapy, Concepts, Current Trials And Future Directions," Ann. Rev. Pharmacol. Toxicol. 32:573-596; Mulligan (1993) "The Basic Science Of Gene Therapy," Science 260:926-932; and Morgan et al. (1993) "Human gene therapy," Ann. Rev. Biochem. 62:191-217). Methods commonly known in the art of recombinant DNA technology which can be used are described in Ausubel et al. (eds.), 1993, CURRENT PROTOCOLS IN MOLECULAR BIOLOGY, John Wiley & Sons, NY; Kriegler, 1990, GENE TRANSFER AND EXPRESSION, A LABORATORY MANUAL, Stockton Press, NY; and in Chapters 12 and 13, Dracopoli et al. (eds), 1994, CURRENT PROTOCOLS IN HUMAN GENETICS, John Wiley & Sons, NY.; Colbere-Garapin et al. (1981) "A New Dominant Hybrid Selective Marker For Higher Eukaryotic Cells," J. Mol. Biol. 150:1-14; and hygro, which confers resistance to hygromycin (Santerre et al. (1984) "Expression Of Prokaryotic Genes For Hygromycin B And G418 Resistance As Dominant-Selection Markers In Mouse L Cells," Gene 30:147-156).

The expression levels of an antibody of the disclosure can be increased by vector amplification (for a review, see Bebbington and Hentschel, "The Use Of Vectors Based On Gene Amplification For The Expression Of Cloned Genes In Mammaian Cells," in DNA CLONING, Vol. 3. (Academic Press, New York, 1987)). When a marker in the vector system expressing an antibody is amplifiable, increase in the level of inhibitor present in culture of host cell will increase the number of copies of the marker gene. Since the amplified region is associated with the nucleotide sequence of the antibody, production of the antibody will also increase (Crouse et al. (1983) "Expression And Amplification Of Engineered Mouse Dihydrofolate Reductase Minigenes," Mol. Cell. Biol. 3:257-266).

The host cell may be co-transfected with two expression vectors of the disclosure , the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot (1986) "Expression And Amplification Of Engineered Mouse Dihydrofolate Reductase Minigenes," Nature 322:562-565; Kohler (1980) "Immunoglobulin Chain Loss In Hybridoma Lines," Proc. Natl. Acad. Sci. (U.S.A.) 77:2197-2199). The coding sequences for the heavy and light chains may comprise cDNA or genomic DNA.

In general, glycoproteins produced in a particular cell line or transgenic animal will have a glycosylation pattern that is characteristic for glycoproteins produced in the cell line or transgenic animal. Therefore, the particular glycosylation pattern of an antibody will depend on the particular cell line or transgenic animal used to produce the antibody. However, all antibodies encoded by the nucleic acid molecules provided herein, or comprising the amino acid sequences provided herein, comprise the instant disclosure, independent of the glycosylation pattern the antibodies may have. Similarly, in particular embodiments, antibodies with a glycosylation pattern comprising only non-fucosylated N-glycans may be advantageous, because these antibodies have been shown to typically exhibit more potent efficacy than their fucosylated counterparts both *in vitro* and *in vivo (See* for example, Shinkawa et al., J. Biol. Chem. 278: 3466-3473 (2003); U.S. Patent Nos. 6,946,292 and 7,214,775). These antibodies with non-fucosylated *N*-glycans are not likely to be immunogenic because their carbohydrate structures are a normal component of the population that exists in human serum IgG.

Once the antibody of the disclosure has been recombinantly expressed, it may be purified by any method known in the art for purification of an antibody, for example, by chromatography (e.g., ion exchange, affinity, particularly by affinity for the specific antigen after Protein A, and sizing column chromatography), centrifugation, differential solubility, or by any other standard technique for the purification of proteins.

Diagnosis of Acute Renal Failure

The present invention relates in part to administration of anti-TIMP-2 antibodies to patients diagnosed with AKI. As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times \text{24-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr\ corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Risk Assessment

Because AKI is associated with significant morbidity and mortality, and because no specific treatment is available to reverse AKI, early recognition and management is paramount. Indeed, recognition of patients at risk for AKI, or with possible AKI but prior to clinical manifestations, is likely to result in better outcomes than treating only established AKI. Thus, the present invention relates in part to administration of anti-TIMP-2 antibodies to patients at high risk of imminent (within 72 hours, and more preferably 48, 24, 18, or 12 hours) AKI.

High-risk patients include those with risk factors for acute kidney injury (AKI) but have normal GFR. For example, they may include patients who have diabetes and hypertension or are taking medications such as nonsteroidal anti-inflammatory drugs or angiotensin-converting enzyme inhibitors. These individuals represent a population that needs to be identified to modify risk factors if possible and initiate preventive strategies when indicated (e.g., contrast studies). Patients with prerenal AKI are those with prerenal urinary indices and failure of autoregulatory mechanisms that lead to a decrease in GFR (e.g., dehydration). These individuals have the potential for rapid reversal of their prerenal condition. During this period, injury to brush border of proximal tubule cells may be present but undetectable. However, with novel biomarkers, identification of early injury and treatment is possible. Patients with AKI may represent an extension from severe prerenal AKI. Alternatively, in some conditions, AKI may not be preceded by a prerenal state (e.g., sepsis, exposure to nephrotoxins). Serum creatinine is a late marker and will detect AKI after substantial injury is present. At this point, intervention may be too late.

The KDIGO Clinical Practice Guideline for Acute Kidney Injury (Kidney Intl. 2 (Suppl 1), 2012

provides a description of risk assessment for AKI, particularly in Chapter 2.2 and Appendix D. Risk factors include hydration state, hypoalbuminemia, advanced age, female gender, black race, presence of CKD, diabetes, heart disease, sepsis, or pulmonary disease, exposure to certain medications and contrast agents that are nephrotoxic, cardiac surgery, etc.

Use of Biomarkers

Serum creatinine measurement (SCr) is the gold-standard marker for renal function. However, SCr concentrations may be inaccurate in detecting an abrupt decline in renal function, as the functional reserve of the remaining healthy nephrons prevents a significant rise in SCr until 50% of nephrons are lost. Thus, because SCr is a "late" marker of renal injury, even if the SCr-based estimation of renal function is "normal" renal injury may already have begun. Other biomarkers which have been used to assess AKI include serum and urinary Cystatin C, serum and urinary neutrophil gelatinase-associated lipocalin, urinary Kidney Injury Molecule 1, Interleukin-18, Liver-type fatty acid binding protein, and N-acetyl-$\beta$-D-glucosaminidase.

In 2014, the US Food and Drug Administration approved the marketing of a test based on the combination of urine concentrations of tissue inhibitor of metalloproteinase 2 and insulin-like growth factor binding protein 7 ([TIMP-2] $\times$ [IGFBP7]) to identify patients are at risk for developing future AKI. In one study, a single measurement of urinary TIMP2•IGFBP7 soon after ICU admission and found an AUC-ROC of 0.84 for development of moderate-to-severe AKI within 12 hours. Using a cutoff value of 0.3, the test showed a sensitivity and specificity of 89% (77-97%) and 49% (43-54%), respectively; at a cutoff value of 2.0 the test showed a sensitivity and specificity of 40% (23-57%) and 94% (92-96%). Gunnerson et al., J. Trauma Acute Care Surg. 80: 243-49, 2016. Such tests provide an ideal method of identifying high risk patients for treatment according to the present invention.

Pharmaceutical Compositions and Administration

To prepare pharmaceutical or sterile compositions of the anti-TIMP-2 antibodies and antigen-binding fragments of the disclosure , the antibody or antigen-binding fragment thereof is admixed with a pharmaceutically acceptable carrier or excipient. See, *e.g.,* Remington's Pharmaceutical Sciences and U.S. Pharmacopeia: National Formulary, Mack Publishing Company, Easton, PA (1984).

Formulations of therapeutic and diagnostic agents may be prepared by mixing with acceptable carriers, excipients, or stabilizers in the form of, *e.g.,* lyophilized powders, slurries, aqueous solutions or suspensions (see, *e.g.,* Hardman, et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

Toxicity and therapeutic efficacy of the antibodies of the disclosure, administered alone or in combination with another therapeutic agent, can be determined by standard pharmaceutical procedures in cell cultures or experimental animals, *e.g.,* for determining the $LD_{50}$ (the dose lethal to 50% of the population) and the $ED_{50}$ (the dose therapeutically effective in 50% of the population). The dose ratio between toxic and therapeutic effects is the therapeutic index ($LD_{50}/ED_{50}$). The data obtained from these cell culture assays and animal studies can be used in formulating a range of dosage for use in human. The dosage of such compounds lies preferably within a range of circulating concentrations that include the $ED_{50}$ with little or no toxicity. The dosage may vary within this range depending upon the dosage form employed and the route of administration.

In a further embodiment, a further therapeutic agent that is administered to a subject in association with an anti-TIMP-2 antibody or antigen-binding fragment thereof of the disclosure in accordance with the Physicians' Desk Reference 2003 (Thomson Healthcare; 57th edition (November 1, 2002)).

The mode of administration can vary. Routes of administration include oral, rectal, transmucosal, intestinal, parenteral; intramuscular, subcutaneous, intradermal, intramedullary, intrathecal, direct intraventricular, intravenous, intraperitoneal, intranasal, intraocular, inhalation, insufflation, topical, cutaneous, transdermal, or intra-arterial.

In particular embodiments, the anti-TIMP-2 antibodies or antigen-binding fragments thereof of the disclosure can be administered by an invasive route such as by injection. In further embodiments of the invention, an anti-TIMP-2 antibody or antigen-binding fragment thereof, or pharmaceutical composition thereof, is administered intravenously, subcutaneously, intramuscularly, intraarterially, or by inhalation, aerosol delivery. Administration by non-invasive routes (e.g., orally; for example, in a pill, capsule or tablet) is also within the scope of the present invention.

The present disclosure provides a vessel (e.g., a plastic or glass vial, e.g., with a cap or a chromatography column, hollow bore needle or a syringe cylinder) comprising any of the antibodies or antigen-binding fragments of the disclosure or a pharmaceutical composition thereof. The present disclosure also provides an injection device comprising any of the antibodies or antigen-binding fragments of the disclosure or a pharmaceutical composition thereof. An injection device is a device that introduces a substance into the body of a patient via a parenteral route, *e.g.,* intramuscular, subcutaneous or intravenous. For example, an injection device may be a syringe *(e.g.,* pre-filled with the pharmaceutical composition, such as an auto-injector) which, for example, includes a cylinder or barrel for holding fluid to be injected *(e.g.,* antibody or fragment or a pharmaceutical composition thereof), a needle for piecing skin and/or blood vessels for injection of the fluid; and a plunger for pushing the fluid out of the cylinder and through the needle bore. In an instance of the disclosure , an injection device that comprises an antibody or antigen-binding fragment thereof of the present disclosure or a pharmaceutical composition thereof is an intravenous (IV) injection device. Such a device includes the antibody or fragment or a pharmaceutical composition thereof in a cannula or trocar/needle which may be attached to a tube which may be attached to a bag or reservoir for holding fluid (e.g., saline; or lactated ringer solution comprising NaCl, sodium lactate, KCl, $CaCl_2$ and optionally including glucose) introduced into the body of the patient through the cannula or trocar/needle. The antibody or fragment or a pharmaceutical composition thereof may, in an instance of the disclosure , be introduced into the device once the trocar and cannula are inserted into the vein of a subject and the trocar is removed from the inserted cannula. The IV device may, for example, be inserted into a peripheral vein *(e.g.,* in the hand or arm); the superior vena cava or inferior vena cava, or within the right atrium of the heart *(e.g.,* a central IV); or into a subclavian, internal jugular, or a femoral vein and, for example, advanced toward the heart until it reaches the superior vena cava or right atrium *(e.g.,* a central venous line). In an instance of the disclosure, an injection device is an autoinjector; a jet injector or an external infusion pump. A jet injector uses a high-pressure narrow jet of liquid which penetrate the epidermis to introduce the antibody or fragment or a pharmaceutical composition thereof to a patient's body. External infusion pumps are medical devices that deliver the antibody or fragment or a pharmaceutical composition thereof into a patient's body in controlled amounts. External infusion pumps may be powered electrically or mechanically. Different pumps operate in different ways, for example, a syringe pump holds fluid in the reservoir of a syringe, and a moveable piston controls fluid delivery, an elastomeric pump holds fluid in a stretchable balloon reservoir, and pressure from the elastic walls of the balloon drives fluid delivery. In a peristaltic pump, a set of rollers pinches down on a length of flexible tubing, pushing fluid forward. In a multi-channel pump, fluids can be delivered from multiple reservoirs at multiple rates.

The pharmaceutical compositions disclosed herein may also be administered with a needleless hypodermic injection device; such as the devices disclosed in U.S. Patent Nos. 6,620,135; 6,096,002; 5,399,163; 5,383,851; 5,312,335; 5,064,413; 4,941,880; 4,790,824 or 4,596,556. Such needleless devices comprising the pharmaceutical composition are also part of the present disclosure. The pharmaceutical compositions disclosed herein may also be administered by infusion. Examples of well-known implants and modules for administering the pharmaceutical compositions include those disclosed in: U.S. Patent No. 4,487,603, which discloses an implantable micro-infusion pump for dispensing medication at a controlled rate; U.S. Patent No. 4,447,233, which discloses a medication infusion pump for delivering medication at a precise infusion rate; U.S. Patent. No. 4,447,224, which discloses a variable flow implantable infusion apparatus for continuous drug delivery; U.S. Patent. No. 4,439,196, which discloses an osmotic drug delivery system having multi-chamber compartments. Many other such implants, delivery systems, and modules are well known to those skilled in the art and those comprising the pharmaceutical compositions of the present disclosure are within the scope of the present disclosure .

The administration regimen depends on several factors, including the serum or tissue turnover rate of the therapeutic antibody or antigen-binding fragment, the level of symptoms, the immunogenicity of the therapeutic antibody, and the accessibility of the target cells in the biological matrix. Preferably, the administration regimen delivers sufficient therapeutic antibody or fragment to effect improvement in the target disease state, while simultaneously minimizing undesired side effects. Accordingly, the amount of biologic delivered depends in part on the particular therapeutic antibody and the severity of the condition being treated. Guidance in selecting appropriate doses of therapeutic antibodies or fragments is available (see, *e.g.,* Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert, et al. (2003) New Engl. J. Med. 348:601-608; Milgrom et al. (1999) New Engl. J. Med. 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343:1594-1602).

Determination of the appropriate dose is made by the clinician, e.g., using parameters or factors known or suspected in the art to affect treatment. Generally, the dose begins with an amount somewhat less than the optimum dose and it is increased by small increments thereafter until the desired or optimum effect is achieved relative to any negative side effects. Important diagnostic measures include those of symptoms of, *e.g.,* the inflammation or level of inflammatory cytokines produced. In general, it is desirable that a biologic that will be used is derived from the same species as the animal targeted for treatment, thereby minimizing any immune response to the reagent. In the case of human subjects, for example, humanized and fully human antibodies are may be desirable.

Antibodies or antigen-binding fragments thereof disclosed herein may be provided by continuous infusion, or by doses administered, e.g., daily, 1-7 times per week, weekly, bi-weekly, monthly, bimonthly, quarterly, semiannually, annually etc. Doses may be provided, e.g., intravenously, subcutaneously, topically, orally, nasally, rectally, intramuscular, intracerebrally, intraspinally, or by inhalation. A total weekly dose is generally at least 0.05 $\mu$g/kg body weight, more generally at least 0.2 $\mu$g/kg, 0.5 $\mu$g/kg, 1 $\mu$g/kg, 10 $\mu$g/kg, 100 $\mu$g/kg, 0.25 mg/kg, 1.0 mg/kg, 2.0 mg/kg, 5.0 mg/ml, 10 mg/kg, 25 mg/kg, 50 mg/kg or more (see, *e.g.,* Yang, et al. (2003) New Engl. J. Med. 349:427-434; Herold, et al. (2002) New Engl. J. Med. 346:1692-1698; Liu, et al. (1999) J. Neurol. Neurosurg. Psych. 67:451-456; Portielji, et al. (20003) Cancer Immunol. Immunother. 52:151-144). Doses may also be provided to achieve a pre-determined target concentration of anti-TIMP-2 antibody in the subject's serum, such as 0.1, 0.3, 1, 3, 10, 30, 100, 300 $\mu$g/ml or more. In other embodiments, An anti-TIMP-2 antibody of the present invention is administered, e.g., subcutaneously or intravenously, on a weekly, biweekly, "every 4 weeks," monthly, bimonthly, or quarterly basis at 10, 20, 50, 80, 100, 200, 500, 1000 or 2500 mg/subject.

As used herein, the term "effective amount" refer to an amount of an anti-TIMP-2 or antigen-binding fragment thereof of the invention that, when administered alone or in combination with an additional therapeutic agent to a cell, tissue, or subject, is effective to cause a measurable improvement in one or more symptoms of disease, for example cancer or the progression of cancer. An effective dose further refers to that amount of the antibody or fragment sufficient to result in at least partial amelioration of symptoms, *e.g.,* improved renal function or histology. When applied to an individual active ingredient administered alone, an effective dose refers to that ingredient alone. When applied to a combination, an effective dose refers to combined amounts of the active ingredients that result in the therapeutic effect, whether administered in combination, serially or simultaneously. An effective amount of a therapeutic will result in an improvement of a diagnostic measure or parameter by at least 10%; usually by at least 20%; preferably at least about 30%; more preferably at least 40%, and most preferably by at least 50%. An effective amount can also result in an improvement in a subjective measure in cases where subjective measures are used to assess disease severity.

Additional Treatment Modalities

Once a diagnosis is obtained or a patient is identified as "high risk", the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. These treatments may be used together with the administration of anti-TIMP-2 antibodies according to the present invention.

Management and treatment options for AKI are multifactorial and ultimately must be tailored to each specific situation and patient. As previously stated, RRT, whether continuous or intermittent, is a blood purification system that substitutes for kidney function. However, RRT is associated with risks and complications, as discussed above, and evidence suggests that RRT may be an independent risk factor for worse outcomes. From a risk-benefit perspective, RRT should be reserved for those patients deemed most likely to have persistent AKI and/or most likely not to recover. In this dichotomous construct, conservative management frequently represents the preferred clinical choice for many common clinical scenarios, including situations where clinical evidence, bedside evaluation, and objective data suggest a high likelihood for recovery from and/or non-persistence of AKI.

Conservative management is defined as medical interventions and approaches that address the dangerous and life-threatening manifestations of AKI without the use of RRT. These management strategies include - but are not limited to - the key physiological and pathophysiological disturbances such as hypervolemia and fluid imbalances, acidosis and acid-base disorders, electrolyte disturbances (e.g. hyperkalemia), and uremia and severe azotemia. Conservative management strategies consist of the following basic strategies:
Hemodynamic support:

a. Fluid therapy, including the use of crystalloids, and colloids

b. Administration of vasopressor and vasoactive agents (commonly used vasopressor agents include the following: Norepinephrine, Epinephrine, Phenylephrine, Dopamine, Vasopressin)

c. Diuretic therapy, including the use of loop and osmotic diuretics

d. Treatment of acidosis and other acid-base abnormalities

e. Treatment of potassium and other electrolyte disturbances and imbalances.

f. Glycemic control and treatment of hyperglycemia.

g. Nutritional support.

Hyperkalemia, when severe, is a medical emergency and requires immediate intervention. Treatment approaches are divided into three main approaches: 1) Interventions that cause potassium to shift from the extracellular space into the intracellular space, 2) Interventions that stabilize membrane actions of potassium, and 3) Interventions that enhance potassium elimination. These interventions are often provided concurrently.

Patients with AKI commonly develop acid-base abnormalities, most notably metabolic acidosis, which requires intervention when clinically significant.

Elevated glucose due to stress hyperglycemia is frequently encountered in AKI and other critical illnesses, and while evidence remains controversial with respect to outcome benefit associated glycemic control, patients and populations likely to benefit, and desired glucose targets and the specific therapeutic approaches to achieve (relative) euglycemia, consensus and current standard of care suggest the following: use of insulin therapy to achieve a serum glucose level between 110 to 149 mg/dL (6.1 - 8.3 mmol/L) and above; and frequent monitoring of serum glucose.

Beyond conservative management, physicians may determine that initiation or maintenance of renal replacement therapy is appropriate. Intermittent RRT includes hemodialysis and sustained low-efficiency dialysis, while continuous RRT refers to ultrafiltration (UF), Continuous Venovenous Hemofiltration (CVVH), Continuous Venovenous Hemodialysis (CVVHD), Continuous Venovenous Hemodiafiltration (CVVHDF), Continous Venovenous High-Flux Hemodialysis, and Continuous Arterial Venous Hemofiltration (CAVH).

General Methods

Standard methods in molecular biology are described Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA). Standard methods also appear in Ausbel, et al. (2001) Current Protocols in Molecular Biology, Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described (Coligan, et al. (2000) Current Protocols in Protein Science, Vol. 1, John Wiley and Sons, Inc., New York). Chemical analysis, chemical modification, post-translational modification, production of fusion proteins, glycosylation of proteins are described (see, *e.g.,* Coligan, et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391). Production, purification, and fragmentation of polyclonal and monoclonal antibodies are described (Coligan, et al. (2001) Current Protcols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra).* Standard techniques for characterizing ligand/receptor interactions are available (see, *e.g.,* Coligan, et al. (2001) Current Protocols in Immunology, Vol. 4, John Wiley, Inc., New York).

Monoclonal, polyclonal, and humanized antibodies can be prepared (see, *e.g.,* Sheperd and Dean (eds.) (2000) Monoclonal Antibodies, Oxford Univ. Press, New York, NY; Kontermann and Dubel (eds.) (2001) Antibody Engineering, Springer-Verlag, New York; Harlow and Lane (1988) Antibodies A Laboratory Manual, Cold Spring Harbor Laboratory Press, Cold Spring Harbor, NY, pp. 139-243; Carpenter, et al. (2000) J. Immunol. 165:6205; He, et al. (1998) J. Immunol. 160:1029; Tang et al. (1999) J. Biol. Chem. 274:27371-27378; Baca et al. (1997) J. Biol. Chem. 272:10678-10684; Chothia et al. (1989) Nature 342:877-883; Foote and Winter (1992) J. Mol. Biol. 224:487-499; U.S. Pat. No. 6,329,511).

An alternative to humanization is to use human antibody libraries displayed on phage or human antibody libraries in transgenic mice (Vaughan et al. (1996) Nature Biotechnol. 14:309-314; Barbas (1995) Nature Medicine 1:837-839; Mendez et al. (1997) Nature Genetics 15:146-156; Hoogenboom and Chames (2000) Immunol. Today 21:371-377; Barbas et al. (2001) Phage Display: A Laboratory Manual, Cold Spring Harbor Laboratory Press, Cold Spring Harbor, New York; Kay et al. (1996) Phage Display of Peptides and Proteins: A Laboratory Manual, Academic Press, San Diego, CA; de Bruin et al. (1999) Nature Biotechnol. 17:397-399).

Single chain antibodies and diabodies are described (see, *e.g.,* Malecki et al. (2002) Proc. Natl. Acad. Sci. USA 99:213-218; Conrath et al. (2001) J. Biol. Chem. 276:7346-7350; Desmyter et al. (2001) J. Biol. Chem. 276:26285-26290;

Hudson and Kortt (1999) J. Immunol. Methods 231:177-189; and U.S. Pat. No. 4,946,778). Bifunctional antibodies are provided (see, *e.g.,* Mack, et al. (1995) Proc. Natl. Acad. Sci. USA 92:7021-7025; Carter (2001) J. Immunol. Methods 248:7-15; Volkel, et al. (2001) Protein Engineering 14:815-823; Segal, et al. (2001) J. Immunol. Methods 248:1-6; Brennan, et al. (1985) Science 229:81-83; Raso, et al. (1997) J. Biol. Chem. 272:27623; Morrison (1985) Science 229:1202-1207; Traunecker, et al. (1991) EMBO J. 10:3655-3659; and U.S. Pat. Nos. 5,932,448, 5,532,210, and 6,129,914).

Multispecific antibodies are also provided (see, *e.g.,* Azzoni et al. (1998) J. Immunol. 161:3493; Kita et al. (1999) J. Immunol. 162:6901; Merchant et al. (2000) J. Biol. Chem. 74:9115; Pandey et al. (2000) J. Biol. Chem. 275:38633; Zheng et al. (2001) J. Biol Chem. 276:12999; Propst et al. (2000) J. Immunol. 165:2214; Long (1999) Ann. Rev. Immunol. 17:875); Labrijn et al., Proc. Natl. Acad. Sci. USA 110: 5145-50, 2013; de Jong et al., PLOS Biol 14(1): e1002344, 2016 (doi:10.1371/journal.pbio.1002344).

Purification of antigen is not necessary for the generation of antibodies. Animals can be immunized with cells bearing the antigen of interest. Splenocytes can then be isolated from the immunized animals, and the splenocytes can be fused with a myeloma cell line to produce a hybridoma (see, *e.g.,* Meyaard et al. (1997) Immunity 7:283-290; Wright et al. (2000) Immunity 13:233-242; Preston *et al., supra;* Kaithamana et al. (1999) J. Immunol. 163:5157-5164).

Antibodies can be conjugated, e.g., to small drug molecules, enzymes, liposomes, polyethylene glycol (PEG). Antibodies are useful for therapeutic, diagnostic, kit or other purposes, and include antibodies coupled, *e.g.,* to dyes, radioisotopes, enzymes, or metals, *e.g.,* colloidal gold (see, *e.g.,* Le Doussal et al. (1991) J. Immunol. 146:169-175; Gibellini et al. (1998) J. Immunol. 160:3891-3898; Hsing and Bishop (1999) J. Immunol. 162:2804-2811; Everts et al. (2002) J. Immunol. 168:883-889).

Methods for flow cytometry, including fluorescence activated cell sorting (FACS), are available (see, *e.g.,* Owens, et al. (1994) Flow Cytometry Principles for Clinical Laboratory Practice, John Wiley and Sons, Hoboken, NJ; Givan (2001) Flow Cytometry, 2nd ed.; Wiley-Liss, Hoboken, NJ; Shapiro (2003) Practical Flow Cytometry, John Wiley and Sons, Hoboken, NJ). Fluorescent reagents suitable for modifying nucleic acids, including nucleic acid primers and probes, polypeptides, and antibodies, for use, *e.g.,* as diagnostic reagents, are available (Molecular Probes (2003) Catalogue, Molecular Probes, Inc., Eugene, OR; Sigma-Aldrich (2003) Catalogue, St. Louis, MO).

Standard methods of histology of the immune system are described (see, *e.g.,* Muller-Harmelink (ed.) (1986) Human Thymus: Histopathology and Pathology, Springer Verlag, New York, NY; Hiatt, et al. (2000) Color Atlas of Histology, Lippincott, Williams, and Wilkins, Phila, PA; Louis, et al. (2002) Basic Histology: Text and Atlas, McGraw-Hill, New York, NY).

Software packages and databases for determining, *e.g.,* antigenic fragments, leader sequences, protein folding, functional domains, glycosylation sites, and sequence alignments, are available (see, *e.g.,* GenBank, Vector NTI® Suite (Informax, Inc, Bethesda, MD); GCG Wisconsin Package (Accelrys, Inc., San Diego, CA); DeCypher® (TimeLogic Corp., Crystal Bay, Nevada); Menne, et al. (2000) Bioinformatics 16: 741-742; Menne, et al. (2000) Bioinformatics Applications Note 16:741-742; Wren, et al. (2002) Comput. Methods Programs Biomed. 68:177-181; von Heijne (1983) Eur. J. Biochem. 133:17-21; von Heijne (1986) Nucleic Acids Res. 14:4683-4690).

## EXAMPLES

Example 1: Monoclonal Antibody development in rabbits

Female New Zealand Rabbits are immunized by subcutaneous injections (SQ) with antigen/adjuvant emulsions. Primary immunization is done with Complete Freund's Adjuvant and Incomplete Freund's Adjuvant is used for all subsequent boosts. Rabbits are injected SQ every three weeks at 250μg protein antigen per rabbit (alternating two sites, hips and scapulas). A test bleed is taken from the marginal ear vein seven days after the second boost. This test bleed (immune sera) is tested by indirect ELISA assay to determine if immune response of the rabbit is adequate for monoclonal antibody development. The best responding rabbit is given a final SQ boost and four days later is euthanized via exsanguination. The whole blood is collected via cardiac puncture. B cells producing antibody of interest are identified by indirect ELISA on target antigen and immunoglobulin genes are isolated. Heavy and light chains are cloned into separate mammalian expression vectors, transfected into HEK cells (transient transfection), and tissue culture supernatant containing rabbit monoclonal antibodies are harvested.

Example 2: Monoclonal Antibody development in mice

Female BALB/c mice (60 days old) are immunized by intraperitoneal injections (IP) with antigen/adjuvant emulsions as per standard operating procedure. Primary immunization is done with Complete Freund's Adjuvant and Incomplete Freund's Adjuvant is used for all subsequent boosts. Mice are injected IP every 3 weeks at 25μg antigen per mouse (total volume 125μL per mouse). Test bleeds are done by saphenous vein lancing 7 to 10 days after the second boost.

This test bleed (immune sera) is tested by indirect ELISA assay to determine if the immune response of mice is adequate for fusion. The best 2 responding mice are given a final intravenous boost of $10\mu g$ antigen per mouse in sterile saline via lateral tail vein. 4 days after the IV boost the mice are euthanized and the spleens are harvested. Lymphocytes isolated from the spleen are used in the fusion process to produce hybridomas using the method of Kohler, G.; Milstein, C. (1975). "Continuous cultures of fused cells secreting antibody of predefined specificity". Nature 256 (5517): 495-497. Hybridomas are generated using a PEG1500 fusion process.

Example 3: Humanization

By comparing the murine variable (V) region framework (FR) sequences of the mouse monoclonal to that of human antibodies in the Kabat data base (Kabat et al., Sequences of Proteins of Immunological Interest, 5th ed., U.S. Department of Health and Human Services, U.S. Government Printing Office, Ishington, D.C.), the human sequences are found to exhibit the highest degree of sequence homology to the FRs of VK and VH domains of the mouse monoclonal. Therefore, the these human sequences are selected as the human frameworks onto which the CDRs for the mouse monoclonal are grafted.

Example 4. Histological Evaluations

The standard for determination of kidney injury in an animal model is careful microscopic examination of kidney morphology by a trained pathologist. See, e.g., FDA "Review of Qualification Data for Biomarkers of Nephrotoxicity Submitted by the Predictive Safety Testing Consortium, 2009, page 13 ("Histopathology was used as the gold standard that defined injury"); see also, Vaidya et al., Nat. Biotechnol. 28: 478-85, 2010 (owing to the poor sensitivity and specificity of SCr and BUN, kidney tubular damage is scored by histopathology). The Critical Path Institute's Predictive Safety Testing Consortium (PSTC) Nephrotoxicity Working Group (NWG) created a histopathology lexicon and assigned a severity score grading scale of 0-5 to grade pathological lesions from 0 (no observable pathology), 1 (minimal), 2 (slight), 3 (moderate), 4 (marked) or 5 (severe). This grading scale was used to assess the effect of treatment with anti-TIMP-2.

Example 5:

Balb/c mice (20-24 weeks old, weight 25-30g) were purchased from Charles River Laboratories International, Inc. All procedures were approved by the Institutional Animal Care and Use Committee (IACUC) of the University of Pittsburgh Medical Center (UPMC). After an acclimation period of 1 week, mice were subjected to a cecal ligation and puncture surgery (CLP) to induce sepsis or laparotomy only (Sham) after anesthetized with isoflurane. Specifically, the cecum was exteriorized, ligated at 1/4 from the top, and punctured through 3 times with a 21-gage needle. A droplet of feces was extruded and the ligated and punctured cecum was replaced into the abdomen, and the abdominal wall closed. As standard post-surgical treatment, a one-time administration of fluid resuscitation (saline 40ml/kg) was performed into the scruff of the neck subcutaneously, Buprenorphine (0.05 mg/kg) injected, and antibiotics including Ceftriaxone (25mg/kg) and Metronidazole (12.5mg/kg) intraperitoneally applied immediately after surgery and every 12 hrs for 3 days. All animals were closely monitored and allowed free access to food and water during recovery. Mice were sacrificed 48 hours post the surgery and the kidney tissues harvested and blood samples obtained for further measurements.

Two isotypes of anti-TIMP2 antibodies were provided by Astute Medical, Inc. (San Diego, CA). Antibody TIMP2(NB172-77) is a monoclonal F(ab')2, and Antibody TIMP2(NB251-47) is a polyclonal goat IgG. The CLP animals were randomized to three groups eight hours post the CLP surgery: CLP (treated with vehicle placebo), CLP+ intraperitoneal injection with anti-TIMP2 antibody NB172-77, 5mg/kg), CLP+ intraperitoneal injection with anti-TIMP2 antibody NB251-47, 5mg/kg).

Hidney injury was scored by histology in Hematoxylin and eosin (H&E) staining histology tissue images. It included cell infiltration score (0-3) and tubular epithelium injury score (0-5). Specifically, cell infiltration was determined by scoring the proximate number of cells (layers) surrounding tubular and vessel walls (score: 0 = none, 1 = <10 cells (5 layers), 2 = 10 to 20 cells (5 to 10 cell layers), 3 = > 30 cells (>10 cell layers); Injury of the tubular epithelium was scored based on the proportion of tubules that exhibit cellular necrosis, loss of brush border, cast formation, vacuolization, and tubule dilation as follows: 0, none; 1, <10%; 2, 11% to 25%; 3, 26% to 45%; 4, 46% to 75%; and 5, >76%. Serum creatinine (SCr) was measured with Creatinine Assay kit from BioVision (Mountain View, CA).

Statistic test for comparing groups was analyzed using SPSS version 14.0 software (SPSS Inc., Chicago, IL). Paired experimental groups were compared using t-test. Differences were considered significant at $p < 0.05$. Results are presented as the mean $\pm$ SEM.

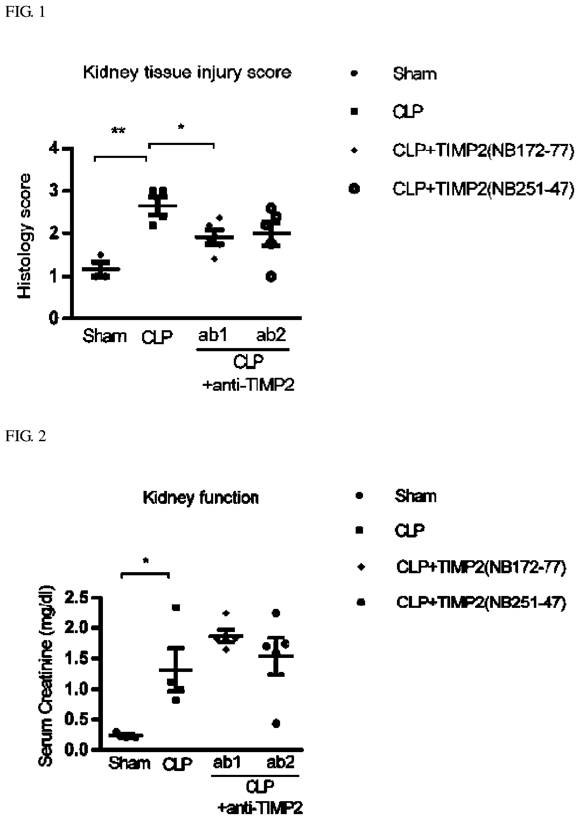

As shown in Fig. 1, anti-TIMP2 treatment with TIMP2(NB 172-77) significantly decreased the extent of kidney tissue injuries compared to untreated sepsis animals (two tailed T-test, P value 0.046). As expected, anti-TIMP2 treatment has no effect on the kidney functions measured by serum creatinine (Fig. 2). Thus, TIMP2 facilitates kidney tissue damage during sepsis independent of serum creatinine level.

The examples provided herein are representative of preferred embodiments, are exemplary, and are not intended as limitations on the scope of the invention.

**Claims**

1. An antibody that specifically binds Metalloproteinase inhibitor 2 (TIMP-2) for use in treating acute kidney injury (AKI) in a subject having or identified as being at increased risk of said acute kidney injury.

2. The antibody for use of claim 1, wherein the subject has chronic kidney disease (CKD) or exhibits one or more symptoms of CKD; or

    wherein the subject has diabetic nephropathy (DN) or exhibits one or more symptoms of DN; or
    wherein the subject has an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, or reduced renal function; or
    wherein the subject is undergoing or has undergone major vascular surgery, coronary artery bypass, or other cardiac surgery; or
    wherein the subject has received one or more of NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

3. The antibody for use of claim 1, wherein the subject has acute kidney injury (AKI), optionally wherein the AKI is RIFLE stage R (Risk), I (Injury) or F (Failure).

4. The antibody for use of one of claims 1-3, wherein the subject is characterized as at or below AKIN stage 1 or RIFLE stage R (Risk).

5. The antibody for use of one of claims 1-3, wherein the subject is characterized as at AKIN stage 2 or RIFLE stage I (Injury).

6. The antibody for use of one of claims 1-3, wherein the subject is characterized as at AKIN stage 3 or RIFLE stage F (Failure).

7. The antibody for use of one of claims 1-6, wherein the administering results in an improvement in:

    (i) estimated glomerular filtration rate (eGFR) of the subject;
    (ii) kidney function in the subject, wherein the subject is a human; and/or
    (iii) renal histology in the subject.

8. The antibody for use of one of claims 1-6, wherein the administering reduces the level of serum creatinine in the subject.

9. The antibody for use of one of claims 1-6, wherein the subject is characterized as at increased risk of imminent AKI by a biomarker result, wherein the biomarker result comprises one or more of a measured urinary TIMP-2 concentration and a measured urinary Insulin-like growth factor-binding protein 7 (IGFBP7) concentration.

10. The antibody for use of claim 9, wherein the biomarker result comprises a combined result calculated from a measured urinary TIMP-2 concentration and a measured urinary IGFBP7 concentration.

11. The antibody for use of one of claims 1-10, wherein the antibody is administered parenterally.

12. The antibody for use of claim 11, wherein the antibody is administered intravenously, intraarterially, subcutaneously, or intraperitoneally.

13. The antibody for use of one of claims 1-12, wherein the antibody is an IgG, an Fab fragment, an F(ab')2, or an scFv.

**14.** The antibody for use of one of claims 1-13, wherein the antibody is administered in association with one or more further therapeutic agents or therapeutic procedures indicated for the improvement of kidney function.

**15.** The antibody for use of claim 14, wherein the one or more further therapeutic agents or therapeutic procedures comprise one or more treatments selected from the group consisting of renal replacement therapy, management of fluid overload, administration of a caspase inhibitor, administration of minocycline, administration of a Poly ADP-ribose polymerase inhibitor, administration of an iron chelator, administration of a treatment for sepsis in a subject in need thereof, administration of insulin, administration of erythropoietin, and administration of a vasodilator.

**Patentansprüche**

**1.** Antikörper, der spezifisch den Metalloproteinase-Inhibitor 2 (TIMP-2; *tissue inhibitor of metalloproteinases 2)* zur Verwendung bei der Behandlung der akuten Nierenschädigung (AKI; *acute kidney injury)* bei einem Subjekt bindet, das an genannter akuter Nierenschädigung leidet oder mit einem erhöhten Risiko für die genannte akute Nierenschädigung identifiziert wurde.

**2.** Antikörper zur Verwendung nach Anspruch 1, wobei das Subjekt an einer chronischen Nierenerkrankung (CKD; chronic kidney disease) leidet oder ein oder mehr Symptom(e) der CKD aufweist; oder

wobei das Subjekt an einer diabetischen Nephropathie (DN) leidet oder ein oder mehr Symptom(e) der DN aufweist; oder
wobei das Subjekt eine bestehende Diagnose von einem oder mehr von Folgendem aufweist: kongestiver Herzinsuffizienz, Präeklampsie, Eklampsie, Diabetes mellitus, Hypertonie, Koronararterienerkrankung, Proteinurie, Niereninsuffizienz, glomerulärer Filtration unter dem Normbereich, Zirrhose, Serumkreatinin über dem Normbereich, Sepsis, Nierenfunktionsschädigung, oder reduzierter Nierenfunktion; oder
wobei sich das Subjekt einer schweren Gefäßoperation, Koronararterien-Bypass-Operation oder einer anderen Herzoperation unterzieht oder unterzogen hat; oder
wobei das Subjekt eines oder mehr von Folgendem erhalten hat: NSAIDs, Cyclosporine, Tacrolimus, Aminoglykoside, Foscarnet, Ethylenglycol, Hämoglobin, Myoglobin, Ifosfamid, Schwermetalle, Methotrexat, radiopake Kontrastmittel oder Streptozotocin.

**3.** Antikörper zur Verwendung nach Anspruch 1, wobei das Subjekt an einer akuten Nierenschädigung (AKI) leidet, gegebenenfalls wobei sich die AKI im RIFLE-Stadium R (Risk; Risiko), I (Injury; Schädigung) oder F (Failure; Versagen) befindet.

**4.** Antikörper zur Verwendung nach einem der Ansprüche 1 bis 3, wobei das Subjekt **dadurch gekennzeichnet ist, dass** es sich im oder unter dem AKIN-Stadium 1 oder RIFLE-Stadium R (Risk; Risiko) befindet.

**5.** Antikörper zur Verwendung nach einem der Ansprüche 1 bis 3, wobei das Subjekt **dadurch gekennzeichnet ist, dass** es sich im AKIN-Stadium 2 oder RIFLE-Stadium I (Injury; Schädigung) befindet.

**6.** Antikörper zur Verwendung nach einem der Ansprüche 1 bis 3, wobei das Subjekt **dadurch gekennzeichnet ist, dass** es sich im AKIN-Stadium 3 oder RIFLE-Stadium F (Failure; Versagen) befindet.

**7.** Antikörper zur Verwendung nach einem der Ansprüche 1 bis 6, wobei die Verabreichung zu einer Verbesserung des Folgenden führt:

(i) der geschätzten glomerulären Filtrationsrate (eGFR; estimated GFR) des Subjekts;
(ii) der Nierenfunktion des Subjekts, wobei das Subjekt ein Mensch ist; und/oder
(iii) der Nierenhistologie des Subjekts.

**8.** Antikörper zur Verwendung nach einem der Ansprüche 1 bis 6, wobei die Verabreichung den Serumkreatininspiegel des Subjekts reduziert.

**9.** Antikörper zur Verwendung nach einem der Ansprüche 1 bis 6, wobei das Subjekt **dadurch gekennzeichnet ist, dass** bei ihm laut eines Biomarker-Ergebnisses ein erhöhtes Risiko für eine drohende AKI vorliegt, wobei das Biomarker-Ergebnis eines oder mehr von Folgendem beinhaltet: eine im Urin gemessene TIMP-2-Konzentration und eine im Urin gemessene Konzentration des insulinähnlichen Wachstumsfaktor-Bindungsproteins 7 (IGFBP7; *insulin-like growth factor-binding protein 7).*

10. Antikörper zur Verwendung nach Anspruch 9, wobei das Biomarker-Ergebnis ein kombiniertes Ergebnis beinhaltet, berechnet aus einer im Urin gemessenen TIMP-2-Konzentration und einer im Urin gemessenen IGFBP7-Konzentration.

11. Antikörper zur Verwendung nach einem der Ansprüche 1 bis 10, wobei der Antikörper parenteral verabreicht wird.

12. Antikörper zur Verwendung nach Anspruch 11, wobei der Antikörper intravenös, intraarteriell, subkutan oder intraperitoneal verabreicht wird.

13. Antikörper zur Verwendung nach einem der Ansprüche 1 bis 12, wobei der Antikörper ein IgG, ein Fab-Fragment, ein F(ab')2 oder ein scFv ist.

14. Antikörper zur Verwendung nach einem der Ansprüche 1 bis 13, wobei der Antikörper zusammen mit einem oder mehr weiteren therapeutischen Mittel(n) oder therapeutischen Verfahren verabreicht wird, das/die zur Verbesserung der Nierenfunktion angezeigt ist/sind.

15. Antikörper zur Verwendung nach Anspruch 14, wobei das eine oder mehr weitere therapeutische Mittel oder therapeutische Verfahren eine oder mehr Behandlung(en) umfasst/umfassen, ausgewählt aus der Gruppe, die aus Folgendem besteht: einer Nierenersatztherapie, dem Management der Flüssigkeitsüberladung, der Verabreichung eines Caspase-Inhibitors, Verabreichung von Minocyclin, Verabreichung eines Poly(ADP-ribose)-Polymerase-Inhibitors, Verabreichung eines Eisenchelators, Verabreichung einer Behandlung gegen Sepsis bei einem Subjekt mit Bedarf daran, Verabreichung von Insulin, Verabreichung von Erythropoetin und Verabreichung eines Vasodilatators.


**Revendications**

1. Anticorps qui se lie spécifiquement à l'inhibiteur de métalloprotéinease-2 (TIMP-2), destiné à une utilisation dans le traitement d'une lésion rénale aiguë (LRA) chez un sujet présentant ou identifié comme présentant un risque accru de ladite lésion rénale aiguë.

2. Anticorps destiné à une utilisation selon la revendication 1, le sujet étant atteint d'une maladie rénale chronique (MRC) ou présentant un ou plusieurs symptômes de MRC ; ou

   le sujet étant atteint d'une néphropathie diabétique (ND) ou présentant un ou plusieurs symptômes de ND ; ou
   le sujet ayant reçu un diagnostic d'une ou plusieurs pathologies parmi une insuffisance cardiaque congestive, une pré-éclampsie, une éclampsie, un diabète sucré, une hypertension, une coronaropathie, une protéinurie, une insuffisance rénale, une filtration glomérulaire inférieure à la plage normale, une cirrhose, une créatinine sérique supérieure à la plage normale, un sepsis, une lésion de la fonction rénale, ou une fonction rénale réduite ; ou
   le sujet subissant ou ayant subi une chirurgie vasculaire majeure, un pontage aorto-coronarien, ou une autre chirurgie cardiaque ; ou
   le sujet ayant reçu un ou plusieurs d'AINS, de cyclosporines, du tacrolimus, d'aminoglycosides, du foscarnet, de l'éthylène glycol, de l'hémoglobine, de la myoglobine, de l'ifosfamide, de métaux lourds, du méthotrexate, d'agents de contraste radio-opaques, ou de la streptozotocine.

3. Anticorps destiné à une utilisation selon la revendication 1, le sujet présentant une lésion rénale aiguë (LRA), la LRA étant optionnellement au stade RIFLE R (Risque), I (Lésion) ou F (Insuffisance).

4. Anticorps destiné à une utilisation selon l'une quelconque des revendications 1 à 3, le sujet étant caractérisé comme étant au stade AKIN 1 ou au stade RIFLE R (Risque) ou à un stade inférieur.

5. Anticorps destiné à une utilisation selon l'une quelconque des revendications 1 à 3, le sujet étant caractérisé comme étant au stade AKIN 2 ou au stade RIFLE I (Lésion).

6. Anticorps destiné à une utilisation selon l'une quelconque des revendications 1 à 3, le sujet étant caractérisé comme étant au stade AKIN 3 ou au stade RIFLE F (Insuffisance) .

7. Anticorps destiné à une utilisation selon l'une quelconque des revendications 1 à 6, l'administration entraînant une amélioration :

    (i) du débit de filtration glomérulaire estimé (DFGe) du sujet ;
    (ii) de la fonction rénale chez le sujet, le sujet étant un humain ; et/ou
    (iii) de l'histologie rénale chez le sujet.

8. Anticorps destiné à une utilisation selon l'une quelconque des revendications 1 à 6, l'administration réduisant le taux de créatinine sérique chez le sujet.

9. Anticorps destiné à une utilisation selon l'une quelconque des revendications 1 à 6, le sujet étant caractérisé comme présentant un risque accru de LRA imminente selon un résultat de biomarqueur, le résultat de biomarqueur comprenant une ou plusieurs d'une concentration urinaire mesurée de TIMP-2 et d'une concentration urinaire mesurée de protéine 7 de liaison au facteur de croissance insuline-like (IGFBP7).

10. Anticorps destiné à une utilisation selon la revendication 9, dans lequel le résultat de biomarqueur comprend un résultat combiné calculé à partir d'une concentration urinaire mesurée de TIMP-2 et d'une concentration urinaire mesurée d'IGFBP7.

11. Anticorps destiné à une utilisation selon l'une quelconque des revendications 1 à 10, l'anticorps étant administré par voie parentérale.

12. Anticorps destiné à une utilisation selon la revendication 11, l'anticorps étant administré par voie intraveineuse, intra-artérielle, sous-cutanée ou intrapéritonéale.

13. Anticorps destiné à une utilisation selon l'une quelconque des revendications 1 à 12, l'anticorps étant une IgG, un fragment Fab, un F(ab')2, ou un scFv.

14. Anticorps destiné à une utilisation selon l'une quelconque des revendications 1 à 13, l'anticorps étant administré en association avec un(e) ou plusieurs agents thérapeutiques ou procédures thérapeutiques supplémentaires indiqué(e)s pour l'amélioration de la fonction rénale.

15. Anticorps destiné à une utilisation selon la revendication 14, les un(e) ou plusieurs agents thérapeutiques ou procédures thérapeutiques supplémentaires comprenant un ou plusieurs traitements sélectionnés dans le groupe constitué d'un traitement rénal substitutif, d'une prise en charge de la surcharge liquidienne, de l'administration d'un inhibiteur de la caspase, de l'administration de minocycline, de l'administration d'un inhibiteur de la poly(ADP-ribose) polymérase, de l'administration d'un chélateur de fer, de l'administration d'un traitement contre un sepsis chez un sujet qui en a besoin, de l'administration d'insuline, de l'administration d'érythropoïétine, et de l'administration d'un vasodilatateur.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010048346 A
- WO 2011075744 A
- WO 8801649 A
- US 4946778 A
- US 5260203 A
- WO 9404678 A
- WO 9425591 A
- US 6005079 A
- EP 404097 A
- WO 9311161 A
- WO 9211018 A
- US 5530101 A
- US 5585089 A
- US 5693761 A
- US 5693762 A
- US 6180370 B
- US 5859205 A
- US 5821337 A
- US 6054297 A
- US 6407213 B
- US 004590
- US 810502
- US 5571698 A, Ladner
- US 6057098 A
- WO 9824893 A
- WO 9634096 A
- WO 9633735 A
- US 5413923 A
- US 5625126 A
- US 5633425 A
- US 5569825 A
- US 5661016 A
- US 5545806 A
- US 5814318 A
- US 5939598 A
- US 5807715 A
- US 6946292 B
- US 7214775 B
- US 6620135 B
- US 6096002 A
- US 5399163 A
- US 5383851 A
- US 5312335 A
- US 5064413 A
- US 4941880 A
- US 4790824 A
- US 4596556 A
- US 4487603 A
- US 4447233 A
- US 4447224 A
- US 4439196 A
- US 6329511 B
- US 5932448 A
- US 5532210 A
- US 6129914 A

### Non-patent literature cited in the description

- **BELLOMO et al.** *Crit Care,* 2004, vol. 8 (4), R204-12
- **KELLUM.** *Crit. Care Med.,* 2008, vol. 36, 141-45
- **RICCI et al.** *Kidney Int,* 2008, vol. 73, 538-546
- **AKI. JO et al.** *Clin J Am Soc Nephrol,* 2007, vol. 2, 356-365
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **PLUCKTHUN.** THE PHARMACOLOGY OF MONO-CLONAL ANTIBODIES. Springer-Verlag, 1994, vol. 113, 269-315
- **MUYLDERMANS et al.** *Trends Biochem. Sci.,* 2001, vol. 26, 230
- **REICHMANN et al.** *J. Immunol. Methods,* 1999, vol. 231, 25
- **HOLLIGER et al.** *Proc. Natl. Acad. Sci. USA,* 1993, vol. 90, 6444-6448
- **HOLLIGER ; HUDSON.** *Nat. Biotechnol.,* 2005, vol. 23, 1126-1136
- **ERP et al.** *J. Immunoassay,* 1991, vol. 12, 425-43
- *Nelson and Griswold, Comput. Methods Programs Biomed.,* 1988, vol. 27, 65-8
- **JONES.** *Nature,* 1986, vol. 321, 522-525
- **VERHOEYEN et al.** *Science,* 1988, vol. 239, 1534-1536
- **ROQUE et al.** *Biotechnol. Prog.,* 2004, vol. 20, 639-654

- Humanization of Monoclonal Antibodies. **TSURUSHITA ; VASQUEZ.** Molecular Biology of B Cells. Elsevier Science, 2004, 533-545
- **JONES et al.** *Nature,* 1986, vol. 321, 522-525
- **RIECHMANN et al.** *Nature,* 1988, vol. 332, 323-329
- **QUEEN et al.** *Proc Natl Acad Sci, USA,* 1989, vol. 86, 10029-33
- **HE et al.** *J. Immunol.,* 1998, vol. 160, 1029-1035
- **CARTER et al.** *Proc Natl Acad Sci USA,* 1992, vol. 89, 4285-9
- **PRESTA et al.** *Cancer Res.,* 1997, vol. 57 (20), 4593-9
- **GORMAN et al.** *Proc. Natl. Acad. Sci. USA,* 1991, vol. 88, 4181-4185
- **O'CONNOR et al.** *Protein Eng,* 1998, vol. 11, 321-8
- **ROGUSKA et al.** *Proc. Natl. Acad. Sci. USA,* 1994, vol. 91, 969-973
- **WU et al.** *J. Mol. Biol.,* 1999, vol. 294, 151-162
- **BACA et al.** *J. Biol. Chem.,* 1997, vol. 272 (16), 10678-10684
- **ROSOK et al.** *J. Biol. Chem.,* 1996, vol. 271 (37), 22611-22618
- **RADER et al.** *Proc. Natl. Acad. Sci. USA,* 1998, vol. 95, 8910-8915
- **KRAUSS et al.** *Protein Engineering,* 2003, vol. 16 (10), 753-759
- **TAN et al.** *J. Immunol.,* 2002, vol. 169, 1119-1125
- **DE PASCALIS et al.** *J. Immunol.,* 2002, vol. 169, 3076-3084
- **BRUGGEMANN et al.** *Curr Opin Biotechnol,* 1997, vol. 8, 455-458
- **GRIFFITHS et al.** *Curr Opin Biotechnol,* 1998, vol. 9, 102-108
- **HARLOW et al.** ANTIBODIES: A LABORATORY MANUAL. Cold Spring Harbor Laboratory Press, 1988
- **HAMMERLING et al.** MONOCLONAL ANTIBODIES AND T-CELL HYBRIDOMAS. Elsevier, 1981, 563-681
- **ROSSI et al.** *Am. J. Clin. Pathol.,* 2005, vol. 124, 295-302
- **BIEG et al.** GAD65 And Insulin B Chain Peptide (9-23) Are Not Primary Autoantigens In The Type 1 Diabetes Syndrome Of The BB Rat. *Autoimmunity,* 1999, vol. 31 (1), 15-24
- **LODMELL et al.** DNA Vaccination Of Mice Against Rabies Virus: Effects Of The Route Of Vaccination And The Adjuvant Monophosphoryl Lipid A (MPL). *Vaccine,* 2000, vol. 18, 1059-1066
- **JOHNSON et al.** 3-O-Desacyl Monophosphoryl Lipid A Derivatives: Synthesis And Immunostimulant Activities. *Journal of Medicinal Chemistry,* 1999, vol. 42, 4640-4649
- **BALDRIDGE et al.** Monophosphoryl Lipid A (MPL) Formulations For The Next Generation Of Vaccines. *Methods,* 1999, vol. 19, 103-107
- **ETON et al.** Active Immunotherapy With Ultraviolet B-Irradiated Autologous Whole Melanoma Cells Plus DETOX In Patients With Metastatic Melanoma. *Clin. Cancer Res.,* 1998, vol. 4 (3), 619-627
- **GUPTA et al.** Adjuvants For Human Vaccines-Current Status, Problems And Future Prospects. *Vaccine,* 1995, vol. 13 (14), 1263-1276
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **LONBERG et al.** Human Antibodies From Transgenic Mice. *Int. Rev. Immunol.,* 1995, vol. 13, 65-93
- **SAMBROOK et al.** MOLECULAR CLONING, A LABORATORY MANUAL. Cold Spring Harbor Laboratory, 1990
- CURRENT PROTOCOLS IN MOLECULAR BIOLOGY. John Wiley & Sons, 1998
- **RUTHER et al.** Easy Identification Of cDNA Clones. *EMBO J.,* 1983, vol. 2, 1791-1794
- **INOUYE et al.** Up-Promoter Mutations In The Lpp Gene Of Escherichia coli. *Nucleic Acids Res.,* 1985, vol. 13, 3101-3110
- **VAN HEEKE et al.** Expression Of Human Asparagine Synthetase In Escherichia coli. *J. Biol. Chem.,* 1989, vol. 24, 5503-5509
- **LOGAN et al.** Adenovirus Tripartite Leader Sequence Enhances Translation Of mRNAs Late After Infection. *Proc. Natl. Acad. Sci. (U.S.A.),* 1984, vol. 81, 3655-3659
- **BITTER et al.** Expression And Secretion Vectors For Yeast. *Methods in Enzymol.,* 1987, vol. 153, 516-544
- **WIGLER et al.** Transfer Of Purified Herpes Virus Thymidine Kinase Gene To Cultured Mouse Cells. *Cell,* 1977, vol. 11, 223-232
- **SZYBALSKA et al.** Genetics Of Human Cess Line. IV. DNA-Mediated Heritable Transformation Of A Biochemical Trait. *Proc. Natl. Acad. Sci. (U.S.A.),* 1962, vol. 48, 2026-2034
- **LOWY et al.** Isolation Of Transforming DNA: Cloning The Hamster Aprt Gene. *Cell,* 1980, vol. 22, 817-823
- **WIGLER et al.** Transformation Of Mammalian Cells With An Amplfiable Dominant-Acting Gene. *Proc. Natl. Acad. Sci. (U.S.A.),* 1980, vol. 77, 3567-3570
- **O'HARE et al.** Transformation Of Mouse Fibroblasts To Methotrexate Resistance By A Recombinant Plasmid Expressing A Prokaryotic Dihydrofolate Reductase. *Proc. Natl. Acad. Sci. (U.S.A.),* 1981, vol. 78, 1527-1531

- **MULLIGAN et al.** Selection For Animal Cells That Express The Escherichia coli Gene Coding For Xanthine-Guanine Phosphoribosyltransferase. *Proc. Natl. Acad. Sci. (U.S.A.),* 1981, vol. 78, 2072-2076
- **TACHIBANA et al.** Altered Reactivity Of Immunoglobutin Produced By Human-Human Hybridoma Cells Transfected By pSV2-Neo Gene. *Cytotechnology,* 1991, vol. 6 (3), 219-226
- **TOLSTOSHEV.** Gene Therapy, Concepts, Current Trials And Future Directions. *Ann. Rev. Pharmacol. Toxicol.,* 1993, vol. 32, 573-596
- **MULLIGAN.** The Basic Science Of Gene Therapy. *Science,* 1993, vol. 260, 926-932
- **MORGAN et al.** Human gene therapy. *Ann. Rev. Biochem.,* 1993, vol. 62, 191-217
- CURRENT PROTOCOLS IN MOLECULAR BIOLOGY. John Wiley & Sons, 1993
- **KRIEGLER.** GENE TRANSFER AND EXPRESSION, A LABORATORY MANUAL. Stockton Press, 1990
- CURRENT PROTOCOLS IN HUMAN GENETICS. John Wiley & Sons, 1994
- **COLBERE-GARAPIN et al.** A New Dominant Hybrid Selective Marker For Higher Eukaryotic Cells. *J. Mol. Biol.,* 1981, vol. 150, 1-14
- **SANTERRE et al.** Expression Of Prokaryotic Genes For Hygromycin B And G418 Resistance As Dominant-Selection Markers In Mouse L Cells. *Gene,* 1984, vol. 30, 147-156
- The Use Of Vectors Based On Gene Amplification For The Expression Of Cloned Genes In Mammaian Cells. **BEBBINGTON ; HENTSCHEL.** DNA CLONING. Academic Press, 1987, vol. 3
- **CROUSE et al.** Expression And Amplification Of Engineered Mouse Dihydrofolate Reductase Minigenes. *Mol. Cell. Biol.,* 1983, vol. 3, 257-266
- **PROUDFOOT.** Expression And Amplification Of Engineered Mouse Dihydrofolate Reductase Minigenes. *Nature,* 1986, vol. 322, 562-565
- **KOHLER.** Immunoglobulin Chain Loss In Hybridoma Lines. *Proc. Natl. Acad. Sci. (U.S.A.),* 1980, vol. 77, 2197-2199
- **SHINKAWA et al.** *J. Biol. Chem.,* 2003, vol. 278, 3466-3473
- **BAGSHAW et al.** *Nephrol. Dial. Transplant.,* 2008, vol. 23, 1203-1210
- *Kidney Intl,* 2012, vol. 2 (1
- **GUNNERSON et al.** *J. Trauma Acute Care Surg.,* 2016, vol. 80, 243-49
- Remington's Pharmaceutical Sciences and U.S. Pharmacopeia: National Formulary. Mack Publishing Company, 1984
- **HARDMAN et al.** Goodman and Gilman's The Pharmacological Basis of Therapeutics. McGraw-Hill, 2001
- **GENNARO.** Remington: The Science and Practice of Pharmacy. Lippincott, Williams, and Wilkins, 2000
- Pharmaceutical Dosage Forms: Parenteral Medications. Marcel Dekker, 1993
- Pharmaceutical Dosage Forms: Tablets. Marcel Dekker, 1990
- Pharmaceutical Dosage Forms: Disperse Systems. Marcel Dekker, 1990
- **WEINER ; KOTKOSKIE.** Excipient Toxicity and Safety. Marcel Dekker, Inc, 2000
- Physicians' Desk Reference. Thomson Healthcare, 01 November 2002
- **WAWRZYNCZAK.** Antibody Therapy. Bios Scientific Pub. Ltd, 1996
- Monoclonal Antibodies, Cytokines and Arthritis. Marcel Dekker, 1991
- Monoclonal Antibodies and Peptide Therapy in Autoimmune Diseases. Marcel Dekker, 1993
- **BAERT et al.** New Engl. J. Med. 2003, vol. 348, 601-608
- **MILGROM et al.** *New Engl. J. Med.,* 1999, vol. 341, 1966-1973
- **SLAMON et al.** *New Engl. J. Med.,* 2001, vol. 344, 783-792
- **BENIAMINOVITZ et al.** *New Engl. J. Med.,* 2000, vol. 342, 613-619
- **GHOSH et al.** *New Engl. J. Med.,* 2003, vol. 348, 24-32
- **LIPSKY et al.** *New Engl. J. Med.,* 2000, vol. 343, 1594-1602
- **YANG et al.** *New Engl. J. Med.,* 2003, vol. 349, 427-434
- **HEROLD et al.** *New Engl. J. Med.,* 2002, vol. 346, 1692-1698
- **LIU et al.** *J. Neurol. Neurosurg. Psych.,* 1999, vol. 67, 451-456
- **PORTIELJI et al.** *Cancer Immunol. Immunother.,* vol. 52, 151-144
- Merck Manual of Diagnosis and Therapy. Merck Research Laboratories, 1999
- **SAMBROOK ; FRITSCH ; MANIATIS.** Molecular Cloning, A Laboratory Manual. Cold Spring Harbor Laboratory Press
- **SAMBROOK ; RUSSELL.** Molecular Cloning,. Cold Spring Harbor Laboratory Press, 2001
- **WU.** Recombinant DNA. Academic Press, 1993, vol. 217
- **AUSBE et al.** Current Protocols in Molecular Biology. John Wiley and Sons, Inc, 2001, vol. 1-4
- **COLIGAN et al.** Current Protocols in Protein Science. John Wiley and Sons, Inc, 2000, vol. 1
- **COLIGAN et al.** Current Protocols in Protein Science. John Wiley and Sons, 2000, vol. 2
- **AUSUBEL et al.** Current Protocols in Molecular Biology. John Wiley and Sons, Inc, 2001, vol. 3, 16.0.5-16.22.17

- **SIGMA-ALDRICH, CO.** *Products for Life Science Research, St. Louis, MO,* 2001, 45-89
- **AMERSHAM.** Pharmacia Biotech. BioDirectory, 2001, 384-391
- **COLIGAN et al.** Current Protcols in Immunology. John Wiley and Sons, Inc, 2001, vol. 1
- **HARLOW ; LANE.** Using Antibodies. Cold Spring Harbor Laboratory Press, 1999
- **COLIGAN et al.** Current Protocols in Immunology. John Wiley, Inc, 2001, vol. 4
- Monoclonal Antibodies. Oxford Univ. Press, 2000
- Antibody Engineering. Springer-Verlag, 2001
- **HARLOW ; LANE.** Antibodies A Laboratory Manual. Cold Spring Harbor Laboratory Press, 1988, 139-243
- **CARPENTER et al.** *J. Immunol.,* 2000, vol. 165, 6205
- **HE et al.** *J. Immunol.,* 1998, vol. 160, 1029
- **TANG et al.** *J. Biol. Chem.,* 1999, vol. 274, 27371-27378
- **BACA et al.** *J. Biol. Chem.,* 1997, vol. 272, 10678-10684
- **CHOTHIA et al.** *Nature,* 1989, vol. 342, 877-883
- **FOOTE ; WINTER.** *J. Mol. Biol.,* 1992, vol. 224, 487-499
- **VAUGHAN et al.** *Nature Biotechnol.,* 1996, vol. 14, 309-314
- **BARBAS.** *Nature Medicine,* 1995, vol. 1, 837-839
- **MENDEZ et al.** *Nature Genetics,* 1997, vol. 15, 146-156
- **HOOGENBOOM ; CHAMES.** *Immunol. Today,* 2000, vol. 21, 371-377
- **BARBAS et al.** Phage Display: A Laboratory Manual. Cold Spring Harbor Laboratory Press, 2001
- **KAY et al.** Phage Display of Peptides and Proteins: A Laboratory Manual. Academic Press, 1996
- **DE BRUIN et al.** *Nature Biotechnol.,* 1999, vol. 17, 397-399
- **MALECKI et al.** *Proc. Natl. Acad. Sci. USA,* 2002, vol. 99, 213-218
- **CONRATH et al.** *J. Biol. Chem.,* 2001, vol. 276, 7346-7350
- **DESMYTER et al.** *J. Biol. Chem.,* 2001, vol. 276, 26285-26290
- **HUDSON ; KORTT.** *J. Immunol. Methods,* 1999, vol. 231, 177-189
- **MACK et al.** *Proc. Natl. Acad. Sci. USA,* 1995, vol. 92, 7021-7025
- **CARTER.** *J. Immunol. Methods,* 2001, vol. 248, 7-15
- **VOLKEL et al.** *Protein Engineering,* 2001, vol. 14, 815-823
- **SEGAL et al.** *J. Immunol. Methods,* 2001, vol. 248, 1-6
- **BRENNAN et al.** *Science,* 1985, vol. 229, 81-83
- **RASO et al.** *J. Biol. Chem.,* 1997, vol. 272, 27623
- **MORRISON.** *Science,* 1985, vol. 229, 1202-1207
- **TRAUNECKER et al.** *EMBO J.,* 1991, vol. 10, 3655-3659
- **AZZONI et al.** *J. Immunol.,* 1998, vol. 161, 3493
- **KITA et al.** *J. Immunol.,* 1999, vol. 162, 6901
- **MERCHANT et al.** *J. Biol. Chem.,* 2000, vol. 74, 9115
- **PANDEY et al.** *J. Biol. Chem.,* 2000, vol. 275, 38633
- **ZHENG et al.** *J. Biol Chem.,* 2001, vol. 276, 12999
- **PROPST et al.** *J. Immunol.,* 2000, vol. 165, 2214
- **LONG.** *Ann. Rev. Immunol.,* 1999, vol. 17, 875
- **LABRIJN et al.** *Proc. Natl. Acad. Sci. USA,* 2013, vol. 110, 5145-50
- **DE JONG et al.** *PLOS Biol,* 2016, vol. 14 (1), e1002344
- **MEYAARD et al.** *Immunity,* 1997, vol. 7, 283-290
- **WRIGHT et al.** *Immunity,* 2000, vol. 13, 233-242
- **KAITHAMANA et al.** *J. Immunol.,* 1999, vol. 163, 5157-5164
- **LE DOUSSAL et al.** *J. Immunol.,* 1991, vol. 146, 169-175
- **GIBELLINI et al.** *J. Immunol.,* 1998, vol. 160, 3891-3898
- **HSING ; BISHOP.** *J. Immunol.,* 1999, vol. 162, 2804-2811
- **EVERTS ; 2002 et al.** *J. Immunol.,* vol. 168, 883-889
- **OWENS et al.** Flow Cytometry Principles for Clinical Laboratory Practice. John Wiley and Sons, 1994
- **GIVAN.** Flow Cytometry. Wiley-Liss, 2001
- **SHAPIRO.** Practical Flow Cytometry. John Wiley and Sons, 2003
- Molecular Probes. Catalogue. Molecular Probes, Inc, 2003
- **SIGMA-ALDRICH.** *Catalogue,* 2003
- Human Thymus: Histopathology and Pathology. Springer Verlag
- **HIATT et al.** Color Atlas of Histology. Lippincott, Williams, and Wilkins, 2000
- **LOUIS et al.** Basic Histology: Text and Atlas. McGraw-Hill, 2002
- **MENNE et al.** *Bioinformatics,* 2000, vol. 16, 741-742
- **MENNE et al.** *Bioinformatics Applications Note,* 2000, vol. 16, 741-742

- **WREN et al.** *Comput. Methods Programs Biomed.,* 2002, vol. 68, 177-181
- **VON HEIJNE.** *Eur. J. Biochem.,* 1983, vol. 133, 17-21
- **VON HEIJNE.** *Nucleic Acids Res.,* 1986, vol. 14, 4683-4690
- **KOHLER, G. ; MILSTEIN, C.** Continuous cultures of fused cells secreting antibody of predefined specificity. *Nature,* 1975, vol. 256 (5517), 495-497
- Sequences of Proteins of Immunological Interest. **KABAT et al.** Department of Health and Human Services, U.S. Government Printing Office
- **FDA.** Review of Qualification Data for Biomarkers of Nephrotoxicity Submitted. *Predictive Safety Testing Consortium,* 2009, 13
- **VAIDYA et al.** *Nat. Biotechnol.,* 2010, vol. 28, 478-85